# EUROPEAN PATENT SPECIFICATION

(11) **EP 3 800 171 B1**
(45) Date of publication and mention of the grant of the patent: **27.09.2023**
(21) Application number: 20199359.9
(22) Date of filing: 17.03.2016
(51) Int. Cl.: C07C 2/84, C07C 7/11, C07C 7/00, C07C 2/08, C07C 29/151, C01B 3/24, B01J 8/00, C01B 3/38, C01C 1/04, C07C 1/20, C07C 6/04, C07C 51/15, C07C 17/02, C07C 17/10, C07C 2/06, C07D 301/04, C07C 29/10, C10G 50/00, C08F 10/00

(54) **OXIDATIVE COUPLING OF METHANE METHODS AND SYSTEMS**
OXIDATIVE KUPPLUNG VON METHAN, VERFAHREN UND SYSTEME
SYSTÈMES ET PROCÉDÉS DE COUPLAGE OXYDANT DU MÉTHANE

(30) Priority: 17.03.2015 US 201562134508 P; 31.03.2015 US 201562141177 P; 24.04.2015 US 201562152706 P; 01.07.2015 US 201514789953; 08.07.2015 US 201562190182 P; 21.07.2015 US 201562195237 P; 26.02.2016 US 201662300287 P
(43) Date of publication of application: 07.04.2021
(62) Divisional of application: 16765752.7
(73) Proprietor: Lummus Technology LLC, The Woodlands, Texas 77380 (US)
(72) Inventor: RADAELLI, Guido, South San Francisco, CA 94080 (US); BRIDGES, Robert, Friendswood, TX 77546 (US); RAFIQUE, Humera A, Dublin, CA 94568 (US); DUGGAL, Suchia, San Rafael, CA 94903 (US); VUDDAGIRI, Srinivas, Roseville, CA 95661 (US); CIZERON, Joel M, Redwood City, CA 94061 (US); MCCORMICK, Jarod, San Carlos, CA 94070 (US); PATEL, Bipinkumar, Richmond, TX 77407 (US); LAKHAPATRI, Satish, Menlo Park, CA 94025 (US)
(74) Representative: TLIP Limited

(56) References cited:
- US-A- 4 523 049
- US-B2- 7 868 216

## Description

### CROSS-REFERENCE

This application claims the benefit of U.S. Provisional Patent Application No. 62/134,508, filed March 17, 2015, U.S. Provisional Patent Application No. 62/141,177, filed March 31, 2015, U.S. Provisional Patent Application No. 62/152,706, filed April 24, 2015, U.S. Application No. 14/789,953, filed July 1, 2015, U.S. Provisional Patent Application No. 62/190,182, filed July 8, 2015, U.S. Provisional Patent Application No. 62/195,237, filed July 21, 2015, and U.S. Provisional Patent Application No. 62/300,287, filed February 26, 2016.

### BACKGROUND

There exists an infrastructure for chemical production throughout the world. This infrastructure is deployed on virtually every continent, addresses wide ranging industries, and employs a wide variety of different implementations of similar or widely differing technologies. US7868216 describes a process for producing propylene, particularly employing a step for metathesis of 2-butenes by ethylene in which at least a portion of the 2-butenes is produced by dimerising ethylene.
US4523049 describes synthesis of hydrocarbons from a methane source, particularly a method for converting natural gas to more readily transportable material.

### SUMMARY

The present disclosure provides systems and methods for reacting methane in an oxidative coupling of methane ("OCM") process to yield products comprising hydrocarbon compounds with two or more carbon atoms (also "C₂+ compounds" herein). OCM systems and methods of the disclosure can be integrated with various chemical processes, such as methanol (MeOH) production, chlorine (Cl₂) and sodium hydroxide (NaOH) production (e.g., chloralkali process), vinylchloride monomer (VCM) production, ammonia (NH₃) production, processes having syngas (e.g., mixtures of hydrogen (H₂) and carbon monoxide (CO) in any proportion), or olefin derivative production.
A method for producing propylene is the first aspect of the present invention and is provided in claim 1. A system for producing propylene is the second aspect of the present invention and is provided in claim 3. Preferred embodiments are provided in the dependent claims. Any embodiments of the disclosure below which are not encompassed by the claims are provided for reference only.

An aspect of the present disclosure provides a method for oxidative coupling of methane (OCM) to generate hydrocarbon compounds containing at least two carbon atoms (C2+ compounds), comprising: (a) injecting oxygen (O₂), methane (CH₄) and ethane (C₂H₆) into an OCM reactor, wherein the OCM reactor comprises an OCM catalyst for facilitating an OCM reaction, and wherein the C₂H₆ has a concentration of at least about 3 mol% within the OCM catalyst bed; and (b) with the aid of the OCM catalyst in the OCM reactor, performing an OCM reaction to convert the CH4 into C2+ compounds as part of a product stream.

In some embodiments of aspects provided herein, the C₂H₆ has a concentration of at least about 3 mol% at an inlet of the OCM catalyst bed. In some embodiments of aspects provided herein, at least a portion of the C₂H₆ is injected into the OCM reactor separately from the CH₄. In some embodiments of aspects provided herein, the method further comprises increasing or decreasing an amount of CH₄ injected in (a) to maintain the concentration of C₂H₆ within +/- 0.2 mol% during the injecting. In some embodiments of aspects provided herein, the product stream comprises ethane. In some embodiments of aspects provided herein, the method further comprises recycling at least a portion of the ethane in the product stream to the OCM reactor.

An aspect of the present disclosure provides an oxidative coupling of methane (OCM) system for generating hydrocarbon compounds containing at least two carbon atoms (C₂+ compounds), comprising: an OCM reactor that (i) receives oxygen (O₂), methane (CH₄) and ethane (C₂H₆), wherein the C₂H₆ has a concentration of at least about 3 mol% at an inlet of the OCM reactor, and (ii) reacts the CH₄ and O₂ to yield a product stream comprising the C₂₊ compounds.

In some embodiments of aspects provided herein, at least a portion of the C₂H₆ is injected into the OCM reactor separately from the CH₄. In some embodiments of aspects provided herein, the system further comprises a control system that increases or decreases an amount of CH₄ received by the OCM reactor to maintain the concentration of C₂H₆ within +/- 0.2 mol% during the receiving. In some embodiments of aspects provided herein, the product stream further comprises ethane. In some embodiments of aspects provided herein, at least a portion of the ethane in the product stream is recycled to the OCM reactor.

An aspect of the present disclosure provides a method for oxidative coupling of methane (OCM) to generate hydrocarbon compounds containing at least two carbon atoms (C₂+ compounds), comprising: (a) injecting oxygen (O₂), methane (CH₄) and ethane (C₂H₆) into an OCM reactor, wherein the C₂H₆ has a concentration of at least about 3 mol%; and (b) with the aid of an OCM catalyst in the OCM reactor, performing an OCM reaction to convert the CH₄ into C₂₊ compounds as part of a product stream.

In some embodiments of aspects provided herein, at least some of the C₂H₆ is injected into the OCM reactor separately from the CH₄. In some embodiments of aspects provided herein, the method further comprises increasing or decreasing an amount of CH₄ injected in (a) to maintain the concentration of C₂H₆ within +/- 0.2 mol% during the injecting. In some embodiments of aspects provided herein, the product stream comprises ethane, and wherein at least a portion of the ethane in the product stream is recycled to the OCM reactor.

An aspect of the present disclosure provides a method for producing methanol (MeOH) and hydrocarbon compounds containing at least two carbon atoms (C₂+ compounds), comprising: (a) directing methane (CH₄) and oxygen (O₂) into an oxidative coupling of methane (OCM) reactor to produce a product stream comprising the C₂₊ compounds, carbon monoxide (CO) and/or carbon dioxide (CO₂), and un-reacted CH₄; (b) enriching the CO and/or CO₂ from the product stream to generate an enriched CO and/or CO₂ stream; (c) directing the enriched CO and/or CO₂ stream to an MeOH reactor to produce MeOH; (d) enriching the un-reacted CH₄ from the product stream to produce an enriched CH₄ stream; and (e) directing at least a portion of the enriched CH₄ stream to a steam methane reformer (SMR) that produces hydrogen (H₂) and CO and/or CO₂.

In some embodiments of aspects provided herein, the method further comprises directing CO and/or CO₂ produced in the SMR to the MeOH reactor. In some embodiments of aspects provided herein, all of the CO and/or CO₂ from the product stream and all of the CO and/or CO₂ from the SMR is converted to MeOH in the MeOH reactor. In some embodiments of aspects provided herein, the un-reacted CH₄ is provided as fuel to the SMR. In some embodiments of aspects provided herein, the un-reacted CH₄ is provided as feedstock to the SMR, and wherein the SMR converts the un-reacted CH₄ into the H₂ and the at least one of CO and CO₂ for conversion to MeOH in the MeOH reactor. In some embodiments of aspects provided herein, at least about 95% of the methane is converted into MeOH or C₂₊ products. In some embodiments of aspects provided herein, the method further comprises providing the C₂₊ compounds to a cracker that cracks or refines the C₂₊ compounds. In some embodiments of aspects provided herein, at least 80% of the methane consumed by the SMR is from the enriched CH₄ stream. In some embodiments of aspects provided herein, the method further comprises directing a portion of the enriched CH₄ stream to a cracker. In some embodiments of aspects provided herein, at least 80% of the methane consumed by the SMR and the cracker is from the enriched CH₄ stream. In some embodiments of aspects provided herein, the method further comprises directing at least a portion of the enriched CH₄ stream to a methane-consuming process. In some embodiments of aspects provided herein, at least 80% of the methane consumed by the SMR, the cracker and the methane-consuming process is from the enriched CH₄ stream. In some embodiments of aspects provided herein, the product stream comprises CO. In some embodiments of aspects provided herein, the product stream comprises CO₂. In some embodiments of aspects provided herein, the product stream comprises CO and CO₂.

An aspect of the present disclosure provides a system for producing methanol (MeOH) and hydrocarbon compounds containing at least two carbon atoms (C₂+ compounds), comprising: an oxidative coupling of methane (OCM) reactor that (i) receives methane (CH₄) and oxygen (O₂) and (ii) reacts the CH₄ and O₂ to yield a product stream comprising the C₂₊ compounds, carbon monoxide (CO) and/or carbon dioxide (CO₂), and un-reacted CH₄; an MeOH reactor that (i) receives CO and/or CO₂ enriched from the product stream and (ii) reacts the CO and/or CO₂ to produce MeOH; and a steam methane reformer (SMR) that (i) receives un-reacted CH₄ enriched from the product stream and (ii) provides hydrogen (H₂) and at least one of carbon monoxide (CO) and CO₂ to the MeOH reactor to produce MeOH.

In some embodiments of aspects provided herein, the system further comprises a separation unit downstream of the OCM reactor and upstream of the MeOH reactor, wherein the separation unit enriches the CO and/or CO₂ from the product stream. In some embodiments of aspects provided herein, the system further comprises a separation unit downstream of the OCM reactor and upstream of the SMR, wherein the separation unit enriches the un-reacted CH₄ from the product stream. In some embodiments of aspects provided herein, the SMR uses the un-reacted CH₄ as fuel. In some embodiments of aspects provided herein, the SMR uses the un-reacted CH₄ as a feedstock and converts the un-reacted CH₄ into the H₂ and the at least one of CO and CO₂ for conversion to MeOH in the MeOH reactor. In some embodiments of aspects provided herein, the MeOH reactor converts all of the CO₂ from the product stream and all of the CO₂ from the SMR to MeOH. In some embodiments of aspects provided herein, at least about 95% of the methane is converted into MeOH or C₂₊ products. In some embodiments of aspects provided herein, the system further comprises a cracker that (i) receives the C₂₊ compounds and (ii) cracks or refines the C₂₊ compounds. In some embodiments of aspects provided herein, the un-reacted CH₄ directed to the SMR provides at least 80% of the methane consumed by the SMR. In some embodiments of aspects provided herein, the system further comprises a cracker that receives at least a portion of the unreacted CH₄. In some embodiments of aspects provided herein, at least 80% of the methane consumed by the SMR and the cracker is from the unreacted CH₄. In some embodiments of aspects provided herein, the system further comprises a methane-consuming module that receives the enriched CH₄. In some embodiments of aspects provided herein, at least 80% of the methane consumed by the SMR, the cracker and the methane-consuming module is from the unreacted CH₄. In some embodiments of aspects provided herein, the product stream comprises CO. In some embodiments of aspects provided herein, the product stream comprises CO₂. In some embodiments of aspects provided herein, the product stream comprises CO and CO₂.

An aspect of the present disclosure provides a method for producing methanol (MeOH) and hydrocarbon compounds containing at least two carbon atoms (C₂+ compounds), comprising: (a) directing methane (CH₄) and oxygen (O₂) into an oxidative coupling of methane (OCM) reactor to produce a product stream comprising the C₂₊ compounds, carbon monoxide (CO) and/or carbon dioxide (CO₂), and un-reacted CH₄; (b) enriching the CO and/or CO₂ from the product stream to generate an enriched CO and/or CO₂ stream; and (c) directing the enriched CO and/or CO₂ stream to an MeOH reactor to produce MeOH.

In some embodiments of aspects provided herein, all of the CO and/or CO₂ from the product stream is converted to MeOH in the MeOH reactor. In some embodiments of aspects provided herein, at least about 95% of the methane is converted into MeOH or C₂₊ products. In some embodiments of aspects provided herein, the method further comprises providing the C₂₊ compounds to a cracker that cracks or refines the C₂₊ compounds. In some embodiments of aspects provided herein, the product stream comprises CO. In some embodiments of aspects provided herein, the product stream comprises CO₂. In some embodiments of aspects provided herein, the product stream comprises CO and CO₂.

An aspect of the present disclosure provides a system for producing methanol (MeOH) and hydrocarbon compounds containing at least two carbon atoms (C₂+ compounds), comprising: an oxidative coupling of methane (OCM) reactor that (i) receives methane (CH₄) and oxygen (O₂) and (ii) reacts the CH₄ and O₂ to yield a product stream comprising the C₂₊ compounds, carbon monoxide (CO) and/or carbon dioxide (CO₂), and un-reacted CH₄; an MeOH reactor that (i) receives CO and/or CO₂ enriched from the product stream and (ii) reacts the CO and/or CO₂ to produce MeOH.

In some embodiments of aspects provided herein, the MeOH reactor converts all of the CO₂ from the product stream to MeOH. In some embodiments of aspects provided herein, the system further comprises a separation unit downstream of the OCM reactor and upstream of the MeOH reactor, wherein the separation unit enriches the CO and/or CO₂ from the product stream. In some embodiments of aspects provided herein, at least about 95% of the methane is converted into MeOH or C₂₊ products. In some embodiments of aspects provided herein, the system further comprises a cracker that (i) receives the C₂₊ compounds and (ii) cracks or refines the C₂₊ compounds. In some embodiments of aspects provided herein, the system further comprises a cracker that receives at least a portion of the unreacted CH₄. In some embodiments of aspects provided herein, the product stream comprises CO. In some embodiments of aspects provided herein, the product stream comprises CO₂. In some embodiments of aspects provided herein, the product stream comprises CO and CO₂.

An aspect of the present disclosure provides a method for producing methanol (MeOH) and hydrocarbon compounds containing at least two carbon atoms (C₂+ compounds), comprising: (a) directing methane (CH₄) and oxygen (O₂) into an oxidative coupling of methane (OCM) reactor to produce a product stream comprising the C₂₊ compounds and un-reacted CH₄; (b) enriching the un-reacted CH₄ from the product stream to produce an enriched CH₄ stream; (c) directing at least a portion of the enriched CH₄ stream to a steam methane reformer (SMR) that produces hydrogen (H₂) and CO and/or CO₂; and (d) directing the CO and/or CO₂ to an MeOH reactor to produce MeOH.

In some embodiments of aspects provided herein, all of the CO and/or CO₂ from the SMR is converted to MeOH in the MeOH reactor. In some embodiments of aspects provided herein, the un-reacted CH₄ is provided as fuel to the SMR. In some embodiments of aspects provided herein, the un-reacted CH₄ is provided as feedstock to the SMR, and wherein the SMR converts the un-reacted CH₄ into the H₂ and the at least one of CO and CO₂ for conversion to MeOH in the MeOH reactor. In some embodiments of aspects provided herein, at least about 95% of the methane is converted into MeOH or C₂₊ products. In some embodiments of aspects provided herein, the method further comprises providing the C₂₊ compounds to a cracker that cracks or refines the C₂₊ compounds. In some embodiments of aspects provided herein, at least 80% of the methane consumed by the SMR is from the enriched CH₄ stream. In some embodiments of aspects provided herein, the method further comprises directing a portion of the enriched CH₄ stream to a cracker. In some embodiments of aspects provided herein, at least 80% of the methane consumed by the SMR and the cracker is from the enriched CH₄ stream. In some embodiments of aspects provided herein, the method further comprises directing at least a portion of the enriched CH₄ stream to a methane-consuming process. In some embodiments of aspects provided herein, at least 80% of the methane consumed by the SMR, the cracker and the methane-consuming process is from the enriched CH₄ stream. In some embodiments of aspects provided herein, the product stream comprises CO. In some embodiments of aspects provided herein, the product stream comprises CO₂. In some embodiments of aspects provided herein, the product stream comprises CO and CO₂.

An aspect of the present disclosure provides a system for producing methanol (MeOH) and hydrocarbon compounds containing at least two carbon atoms (C₂+ compounds), comprising: an oxidative coupling of methane (OCM) reactor that (i) receives methane (CH₄) and oxygen (O₂) and (ii) reacts the CH₄ and O₂ to yield a product stream comprising the C₂₊ compounds and un-reacted CH₄; a steam methane reformer (SMR) that (i) receives un-reacted CH₄ enriched from the product stream and (ii) provides hydrogen (H₂) and carbon monoxide (CO) and/or CO₂; and an MeOH reactor that (i) receives the CO and/or CO₂ and (ii) reacts the CO and/or CO₂ to produce MeOH.

In some embodiments of aspects provided herein, the system further comprises a separation unit downstream of the OCM reactor and upstream of the SMR, wherein the separation unit enriches the un-reacted CH₄ from the product stream. In some embodiments of aspects provided herein, the SMR uses the un-reacted CH₄ as fuel. In some embodiments of aspects provided herein, the SMR uses the un-reacted CH₄ as a feedstock and converts the un-reacted CH₄ into the H₂ and the CO and/or CO₂ for conversion to MeOH in the MeOH reactor. In some embodiments of aspects provided herein, the MeOH reactor converts all of the CO₂ from the product stream and all of the CO₂ from the SMR to MeOH. In some embodiments of aspects provided herein, at least about 95% of the methane is converted into MeOH or C₂₊ products. In some embodiments of aspects provided herein, the system further comprises a cracker that (i) receives the C₂₊ compounds and (ii) cracks or refines the C₂₊ compounds. In some embodiments of aspects provided herein, the un-reacted CH₄ directed to the SMR provides at least 80% of the methane consumed by the SMR. In some embodiments of aspects provided herein, the system further comprises a cracker that receives at least a portion of the unreacted CH₄. In some embodiments of aspects provided herein, at least 80% of the methane consumed by the SMR and the cracker is from the unreacted CH₄. In some embodiments of aspects provided herein, the system further comprises a methane-consuming module that receives the enriched CH₄. In some embodiments of aspects provided herein, at least 80% of the methane consumed by the SMR, the cracker and the methane-consuming module is from the unreacted CH₄. In some embodiments of aspects provided herein, the product stream comprises CO. In some embodiments of aspects provided herein, the product stream comprises CO₂. In some embodiments of aspects provided herein, the product stream comprises CO and CO₂.

An aspect of the present disclosure provides a method for producing methanol (MeOH) and hydrocarbon compounds containing at least two carbon atoms (C₂+ compounds), comprising: (a) directing methane (CH₄) and oxygen (O₂) into an oxidative coupling of methane (OCM) reactor to produce a product stream comprising the C₂₊ compounds, carbon monoxide (CO) and/or carbon dioxide (CO₂), and un-reacted CH₄; (b) enriching the CO and/or CO₂ from the product stream to generate an enriched CO and/or CO₂ stream that is directed to an MeOH reactor to produce MeOH; and (c) enriching the un-reacted CH₄ from the product stream to produce an enriched CH₄ stream that is directed to a steam methane reformer (SMR), which SMR provides hydrogen (H₂) and at least one of carbon monoxide (CO) and CO₂ to the MeOH reactor to produce MeOH.

In some embodiments of aspects provided herein, the un-reacted CH₄ is provided as fuel to the SMR. In some embodiments of aspects provided herein, the un-reacted CH₄ is provided as feedstock to the SMR, and wherein the SMR converts the un-reacted CH₄ into the H₂ and the at least one of CO and CO₂ for conversion to MeOH in the MeOH reactor. In some embodiments of aspects provided herein, all of the CO₂ from the product stream and all of the CO₂ from the SMR is converted to MeOH in the MeOH reactor. In some embodiments of aspects provided herein, at least about 95% of the methane is converted into MeOH or C₂₊ products. In some embodiments of aspects provided herein, the method further comprises providing the C₂₊ compounds to a cracker that cracks or refines the C₂₊ compounds. In some embodiments of aspects provided herein, the un-reacted CH₄ directed to the SMR provides at least 80% of the methane consumed by the SMR. In some embodiments of aspects provided herein, the method further comprises directing the CH₄ enriched in (c) to a cracker. In some embodiments of aspects provided herein, the un-reacted CH₄ directed to the SMR and the cracker provides at least 80% of the methane consumed by the SMR and the cracker. In some embodiments of aspects provided herein, the method further comprises directing the enriched CH₄ to a methane-consuming process. In some embodiments of aspects provided herein, the un-reacted CH₄ directed to the SMR, the cracker and the methane-consuming process provides at least 80% of the methane consumed by the SMR, the cracker and the methane-consuming process.

An aspect of the present disclosure provides a system for producing methanol (MeOH) and hydrocarbon compounds containing at least two carbon atoms (C₂+ compounds), comprising: an oxidative coupling of methane (OCM) reactor that (i) receives methane (CH₄) and oxygen (O₂) and (ii) reacts the CH₄ and O₂ to yield a product stream comprising the C₂₊ compounds, carbon monoxide (CO) and/or carbon dioxide (CO₂), and un-reacted CH₄; an MeOH reactor that (i) receives CO and/or CO₂ enriched from the product stream and (ii) reacts the CO and/or CO₂ to produce MeOH; and a steam methane reformer (SMR) that (i) receives un-reacted CH₄ enriched from the product stream and (ii) provides hydrogen (H₂) and at least one of carbon monoxide (CO) and CO₂ to the MeOH reactor to produce MeOH.

In some embodiments of aspects provided herein, the system further comprises a separation unit downstream of the OCM reactor and upstream of the MeOH reactor, wherein the separation unit enriches the CO and/or CO₂ from the product stream. In some embodiments of aspects provided herein, the system further comprises a separation unit downstream of the OCM reactor and upstream of the SMR, wherein the separation unit enriches the un-reacted CH₄ from the product stream. In some embodiments of aspects provided herein, the SMR uses the un-reacted CH₄ as fuel. In some embodiments of aspects provided herein, the SMR uses the un-reacted CH₄ as a feedstock and converts the un-reacted CH₄ into the H₂ and the at least one of CO and CO₂ for conversion to MeOH in the MeOH reactor. In some embodiments of aspects provided herein, the MeOH reactor converts all of the CO₂ from the product stream and all of the CO₂ from the SMR to MeOH. In some embodiments of aspects provided herein, at least about 95% of the methane is converted into MeOH or C₂₊ products. In some embodiments of aspects provided herein, the system further comprises a cracker that (i) receives the C₂₊ compounds and (ii) cracks or refines the C₂₊ compounds. In some embodiments of aspects provided herein, the un-reacted CH₄ directed to the SMR provides at least 80% of the methane consumed by the SMR. In some embodiments of aspects provided herein, the system further comprises a cracker that receives at least a portion of the CH₄ enriched in (c). In some embodiments of aspects provided herein, the un-reacted CH₄ directed to the SMR and the cracker provides at least 80% of the methane consumed by the SMR and the cracker. In some embodiments of aspects provided herein, the system further comprises a methane-consuming module that receives the enriched CH₄. In some embodiments of aspects provided herein, the un-reacted CH₄ directed to the SMR, the cracker and the methane-consuming module provides at least 80% of the methane consumed by the SMR, the cracker and the methane-consuming module.

An aspect of the present disclosure provides a method for producing chlorine (Cl₂), sodium hydroxide (NaOH) and compounds containing at least two carbon atoms (C₂+ compounds), comprising: (a) directing sodium chloride (NaCl) and water (H₂O) into a chloralkali module that produces chlorine (Cl₂), NaOH and hydrogen (H₂) from the NaCl and H₂O; (b) directing at least a portion of the H₂ produced in (a) to a methanation module that reacts the H₂ and CO and/or CO₂ to produce CH₄; and (c) directing at least a portion of the CH₄ produced in (b) to an OCM module, which OCM module reacts the CH₄ and O₂ in an OCM process to yield the C₂₊ compounds and heat.

In some embodiments of aspects provided herein, the OCM module includes an OCM reactor with an OCM catalyst that generates the C₂₊ compounds. In some embodiments of aspects provided herein, the OCM module uses the heat to generate electrical power. In some embodiments of aspects provided herein, the OCM module includes a turbine for generating the electrical power. In some embodiments of aspects provided herein, the method further comprises using the electrical power generated by the OCM module to electrochemically generate the Cl₂, NaOH and H₂ from the NaCl and H₂O. In some embodiments of aspects provided herein, at least 80% of electrical power consumed by the chloralkali module is produced by the OCM module. In some embodiments of aspects provided herein, at least a portion of the CO and/or CO₂ is produced in the OCM process in the OCM module. In some embodiments of aspects provided herein, the method further comprises directing at least a portion of the Cl₂ produced by the chloralkali module and at least a portion of the C₂₊ compounds produced by the OCM module into an additional module that reacts the at least the portion of the Cl₂ with the at least the portion of the C₂₊ compounds to produce vinyl chloride monomer (VCM) and/or ethylene dichloride (EDC). In some embodiments of aspects provided herein, the C₂₊ compounds comprise less than about 99% ethylene when reacted by the additional module.

An aspect of the present disclosure provides a system for producing chlorine (Cl₂), sodium hydroxide (NaOH) and compounds containing at least two carbon atoms (C₂₊ compounds), comprising: a chloralkali module that (i) accepts sodium chloride (NaCl) and water (H₂O) and (ii) generates chlorine (Cl₂), NaOH and hydrogen (H₂) from the NaCl and H₂O; a methanation module in fluid communication with the chloralkali module, wherein the methanation module (i) accepts the H₂ from the chloralkali module and carbon monoxide (CO) and/or carbon dioxide (CO₂) and (ii) reacts the H₂ and the CO and/or CO₂ to produce methane (CH₄); and an oxidative coupling of methane (OCM) module in fluid communication with the methanation module, wherein the OCM module (i) accepts the CH₄ from the methanation module and oxygen (O₂) and (ii) reacts the CH₄ and the O₂ in an OCM process to yield the C₂₊ compounds and heat.

In some embodiments of aspects provided herein, the OCM module includes an OCM reactor with an OCM catalyst that generates the C₂₊ compounds. In some embodiments of aspects provided herein, the OCM module uses the heat to generate electrical power. In some embodiments of aspects provided herein, the OCM module includes a turbine for generating the electrical power. In some embodiments of aspects provided herein, the chloralkali module uses the electrical power generated by the OCM module to electrochemically generate the Cl₂, NaOH and H₂ from the NaCl and H₂O. In some embodiments of aspects provided herein, at least 80% of electrical power consumed by the chloralkali module is produced by the OCM module. In some embodiments of aspects provided herein, at least a portion of the CO and/or CO₂ in reacted by the methanation module is produced by the OCM module. In some embodiments of aspects provided herein, the system further comprises an additional module in fluid communication with the OCM module and the chloralkali module, wherein the additional module reacts at least a portion of the Cl₂ produced by the chloralkali module with at least a portion of the C₂₊ compounds produced by the OCM module to produce vinyl chloride monomer (VCM) and ethylene dichloride (EDC). In some embodiments of aspects provided herein, the C₂₊ compounds comprise less than about 99% ethylene when reacted by the additional module.

An aspect of the present disclosure provides a method for producing chlorine (Cl₂), sodium hydroxide (NaOH) and compounds containing at least two carbon atoms (C₂+ compounds), comprising: (a) directing methane (CH₄) and oxygen (O₂) into an oxidative coupling of methane (OCM) module that (i) reacts the CH₄ and O₂ in an OCM process to yield the C₂₊ compounds and heat, and (ii) uses the heat to generate electrical power; (b) directing hydrogen (H₂) and carbon monoxide (CO) and/or carbon dioxide (CO₂) into a methanation module that (i) reacts the H₂ and CO and/or CO₂ to produce CH₄, and (ii) directs at least a portion of the CH₄ produced in the methanation module to the OCM module; and (c) directing NaCl and H₂O into a chloralkali module that (i) uses the electrical power produced by the OCM module to electrochemically generate Cl₂, NaOH and H₂ from the NaCl and H₂O, and (ii) direct at least a portion of the H₂ to the methanation module.

In some embodiments of aspects provided herein, the OCM module includes an OCM reactor with an OCM catalyst that generates the C₂₊ compounds. In some embodiments of aspects provided herein, the OCM module includes a turbine for generating the electrical power. In some embodiments of aspects provided herein, at least 80% of electrical power consumed by the chloralkali module is produced by the OCM module. In some embodiments of aspects provided herein, at least a portion of the CO₂ or the CO in reacted by the methanation module is produced by the OCM module. In some embodiments of aspects provided herein, the method further comprises directing at least a portion of the Cl₂ produced by the chloralkali module and at least a portion of the C₂₊ compounds produced by the OCM module into an additional module that reacts the at least a portion of the Cl₂ with the at least a portion of the C₂₊ compounds to produce vinyl chloride monomer (VCM) and ethylene dichloride (EDC). In some embodiments of aspects provided herein, the C₂₊ compounds comprise less than about 99% ethylene when reacted by the additional module.

An aspect of the present disclosure provides a system for producing chlorine (Cl₂), sodium hydroxide (NaOH) and compounds containing at least two carbon atoms (C₂+ compounds), comprising: an oxidative coupling of methane (OCM) module that (i) accepts methane (CH₄) and oxygen (O₂) and reacts the CH₄ and O₂ in an OCM process that yields the C₂₊ compounds and heat, and (ii) uses the heat to generate electrical power; a methanation module in fluid communication with the OCM module, wherein the methanation module (i) accepts hydrogen (H₂) and carbon monoxide (CO) and/or carbon dioxide (CO₂), (ii) reacts the H₂ and CO and/or CO₂ to produce CH₄, and (iii) directs at least a portion of the CH₄ produced in the methanation module to the OCM module; and a chloralkali module in fluid communication with the methanation module, wherein the chloralkali module (i) accepts NaCl and H₂O, (ii) uses the electrical power produced by the OCM module to electrochemically generate Cl₂, NaOH and H₂ from the NaCl and H₂O, and (iii) directs at least a portion of the H₂ to the methanation module.

In some embodiments of aspects provided herein, the OCM module includes an OCM reactor with an OCM catalyst that generates the C₂₊ compounds. In some embodiments of aspects provided herein, the OCM module includes a turbine for generating the electrical power. In some embodiments of aspects provided herein, at least 80% of electrical power consumed by the chloralkali module is produced by the OCM module. In some embodiments of aspects provided herein, at least a portion of the CO₂ or the CO in reacted by the methanation module is produced by the OCM module. In some embodiments of aspects provided herein, the system further comprises an additional module in fluid communication with the OCM module and the chloralkali module, wherein the additional module reacts at least a portion of the Cl₂ produced by the chloralkali module with at least a portion of the C₂₊ compounds produced by the OCM module to produce vinyl chloride monomer (VCM) and ethylene dichloride (EDC). In some embodiments of aspects provided herein, the C₂₊ compounds comprise less than about 99% ethylene when reacted by the additional module.

An aspect of the present disclosure provides a method for producing ammonia (NH₃) and hydrocarbon compounds containing at least two carbon atoms (C₂+ compounds), comprising: (a) directing methane (CH₄) and oxygen (O₂) into an oxidative coupling of methane (OCM) reactor that reacts the CH₄ and the O₂ in an OCM process to yield an OCM product stream comprising the C₂₊ compounds, hydrogen (H₂), and un-reacted CH₄; (b) directing portion of the un-reacted CH₄ from the OCM product stream to (i) a steam methane reformer (SMR) that reacts H₂O and the portion of the un-reacted CH₄ to yield H₂ and CO and/or CO₂, and/or (ii) a secondary reformer that reacts O₂ and the portion of the un-reacted CH₄ to yield H₂ and CO and/or CO₂; and (c) reacting nitrogen (N₂) and the H₂ produced in (a) and/or (b) to yield ammonia (NH₃).

In some embodiments of aspects provided herein, the method further comprises separating air to produce the N₂ reacted in (c) and the O₂ reacted in (b). In some embodiments of aspects provided herein, a ratio of (i) all nitrogen atoms in the NH₃ produced in (c) to (ii) all nitrogen atoms in N₂ produced upon separating the air is at least about 0.50. In some embodiments of aspects provided herein, in (c), the H₂ produced in (a) is reacted to yield NH₃. In some embodiments of aspects provided herein, in (c), the H₂ produced in (b) is reacted to yield NH₃. In some embodiments of aspects provided herein, (b) comprises (i). In some embodiments of aspects provided herein, (b) comprises (ii). In some embodiments of aspects provided herein, (b) comprises (i) and (ii).

An aspect of the present disclosure provides a system for producing ammonia (NH₃) and hydrocarbon compounds containing at least two carbon atoms (C₂+ compounds), comprising: an oxidative coupling of methane (OCM) reactor receives methane (CH₄) and oxygen (O₂) and reacts the CH₄ and the O₂ in an OCM process to yield an OCM product stream comprising the C₂₊ compounds, hydrogen (H₂), and un-reacted CH₄; at least one of (i) a steam methane reformer (SMR) that receives H₂O and a portion of the un-reacted CH₄ and reacts the H₂O and the portion of the un-reacted CH₄ to yield H₂ and CO and/or CO₂ and (ii) a secondary reformer that receives O₂ and a portion of the un-reacted CH₄ and reacts the O₂ and the portion of the un-reacted CH₄ to yield H₂ and CO and/or CO₂; and an ammonia production module that receives nitrogen (N₂) and the H₂ produced in the SMR and/or the secondary reformer and reacts the N₂ and the H₂ to yield ammonia (NH₃).

In some embodiments of aspects provided herein, the system further comprises an air separation module that separates air to produce the N₂ reacted in the ammonia production module and the O₂ reacted in the SMR or the secondary reformer. In some embodiments of aspects provided herein, a ratio of (i) all nitrogen atoms in the NH₃ produced in the ammonia production module to (ii) all nitrogen atoms in N₂ produced upon separating the air is at least about 0.50. In some embodiments of aspects provided herein, the ammonia production module reacts the H₂ produced in the OCM reactor to yield NH₃. In some embodiments of aspects provided herein, the ammonia production module reacts the H₂ produced in the SMR or the secondary reformer to yield NH₃. In some embodiments of aspects provided herein, the system further comprises the SMR. In some embodiments of aspects provided herein, the system further comprises the secondary reformer. In some embodiments of aspects provided herein, the system further comprises the SMR and the secondary reformer.

An aspect of the present disclosure provides a method for producing ammonia (NH₃) and hydrocarbon compounds containing at least two carbon atoms (C₂+ compounds), comprising: (a) separating air to produce an oxygen stream comprising oxygen (O₂) and a nitrogen stream comprising nitrogen (N₂); (b) directing methane (CH₄) and a first portion of the oxygen stream into an oxidative coupling of methane (OCM) reactor that reacts the CH₄ and the O₂ from the first portion of the oxygen stream in an OCM process to yield an OCM product stream comprising the C₂₊ compounds; (c) reacting hydrogen (H₂) and the N₂ produced in (a) to yield ammonia (NH₃).

In some embodiments of aspects provided herein, the method further comprises converting CH₄ to CO₂ and H₂ in a secondary reformer using a second portion of the oxygen stream. In some embodiments of aspects provided herein, the OCM product stream further comprises un-reacted CH₄ and wherein the CH₄ converted in the secondary reformer comprises at least a portion of the un-reacted CH₄. In some embodiments of aspects provided herein, the H₂ reacted in (c) comprises at least a portion of the H₂ produced in the secondary reformer. In some embodiments of aspects provided herein, a ratio of (i) all nitrogen atoms in the NH₃ produced in (c) to (ii) all nitrogen atoms in N₂ produced in (a) is at least about 0.50.

An aspect of the present disclosure provides a system for producing ammonia (NH₃) and hydrocarbon compounds containing at least two carbon atoms (C₂+ compounds), comprising: an air separation module that separates air to produce a nitrogen stream comprising nitrogen (N₂) and an oxygen stream comprising oxygen (O₂); an oxidative coupling of methane (OCM) module in fluid communication with the air separation module, wherein the OCM module (i) accepts methane (CH₄) and a first portion of the oxygen stream and (ii) reacts the CH₄ and the O₂ from the first portion of the oxygen stream in an OCM process that yields the C₂₊ compounds, carbon monoxide (CO) and/or carbon dioxide (CO₂), hydrogen (H₂), and un-reacted CH₄; and an ammonia production module in fluid communication with the OCM module, wherein the ammonia production module (i) accepts the CO, the H₂, and the un-reacted CH₄ from the OCM module, and (ii) produces NH₃ from the CO, the H₂, and the un-reacted CH₄.

In some embodiments of aspects provided herein, the system further comprises a secondary reformer that accepts CH₄ and a second portion of the oxygen stream and converts the CH₄ and the O₂ from the second portion of the oxygen stream to CO₂ and H₂. In some embodiments of aspects provided herein, the OCM product stream further comprises un-reacted CH₄ and wherein the CH₄ converted in the secondary reformer comprises at least a portion of the un-reacted CH₄. In some embodiments of aspects provided herein, the H₂ accepted in by the ammonia production module comprises at least a portion of the H₂ produced in the secondary reformer. In some embodiments of aspects provided herein, a ratio of (i) all nitrogen atoms in the NH₃ produced in the ammonia production module to (ii) all nitrogen atoms in N₂ produced in the air separation module is at least about 0.50.

An aspect of the present disclosure provides a method for producing ammonia (NH₃) and hydrocarbon compounds containing at least two carbon atoms (C₂+ compounds), comprising: (a) separating air to produce oxygen (O₂) and nitrogen (N₂); (b) directing methane (CH₄) and a first portion of the oxygen (O₂) into an oxidative coupling of methane (OCM) reactor that reacts the CH₄ and the first portion of the O₂ in an OCM process to yield an OCM product stream comprising the C₂₊ compounds, carbon monoxide (CO), hydrogen (H₂), and un-reacted CH₄; (c) directing the CO, the H₂ and the un-reacted CH₄ from the OCM product stream to a steam methane reformer (SMR); (d) in the SMR, converting a first portion of the un-reacted CH₄ to CO and H₂; (e) directing a second portion of the un-reacted CH₄ and a second portion of the O₂ into a secondary reformer that converts the second portion of the un-reacted CH₄ and the second portion of the O₂ to CO₂ and H₂ ; and (f) reacting the N₂ produced in (a) and the H₂ produced in (d) and/or (e) to yield ammonia (NH₃).

In some embodiments of aspects provided herein, the H₂ produced in (d) and (e) is reacted to yield ammonia (NH₃).

An aspect of the present disclosure provides a method for producing ammonia (NH₃) and hydrocarbon compounds containing at least two carbon atoms (C₂₊ compounds), comprising: (a) separating air to produce oxygen (O₂) and nitrogen (N₂); (b) directing methane (CH₄) and a first portion of the oxygen (O₂) into an oxidative coupling of methane (OCM) reactor that reacts the CH₄ and the first portion of the O₂ in an OCM process to yield an OCM product stream comprising the C₂₊ compounds; (c) directing CH₄ and a second portion of the oxygen (O₂) into a secondary reformer to convert CH₄ to CO₂ and H₂; and (e) reacting the H₂ produced in (c) with N₂ to yield ammonia (NH₃).

In some embodiments of aspects provided herein, the H₂ is reacted with at least a portion of the N₂ produced in (a). In some embodiments of aspects provided herein, the OCM product stream further comprises un-reacted CH₄, and wherein the CH₄ directed into the secondary reformer comprises at least a portion of the un-reacted CH₄.

An aspect of the present disclosure provides a method for producing ammonia (NH₃) and hydrocarbon compounds containing at least two carbon atoms (C₂+ compounds), comprising: (a) separating air to produce oxygen (O₂) and nitrogen (N₂); (b) directing methane (CH₄) and a first portion of the oxygen (O₂) into an oxidative coupling of methane (OCM) reactor that reacts the CH₄ and the first portion of the O₂ in an OCM process to yield an OCM product stream comprising the C₂₊ compounds; (c) reacting hydrogen (H₂) and the N₂ produced in (a) to yield ammonia (NH₃).

In some embodiments of aspects provided herein, the method further comprises converting CH₄ to CO₂ and H₂ in a secondary reformer using a second portion of the O₂. In some embodiments of aspects provided herein, the OCM product stream further comprises un-reacted CH₄ and wherein the CH₄ converted in the secondary reformer comprises at least a portion of the un-reacted CH₄. In some embodiments of aspects provided herein, the H₂ reacted in (c) comprises at least a portion of the H₂ produced in the secondary reformer.

An aspect of the present disclosure provides a method for producing ammonia (NH₃) and hydrocarbon compounds containing at least two carbon atoms (C₂+ compounds), comprising: (a) directing methane (CH₄) and oxygen (O₂) into an oxidative coupling of methane (OCM) reactor that reacts the CH₄ and the O₂ in an OCM process to yield a product stream comprising the C₂₊ compounds, carbon monoxide (CO), hydrogen (H₂), and un-reacted CH₄; (b) directing the CO, the H₂ and the un-reacted CH₄ from the product stream to a steam methane reformer (SMR); (c) converting a first portion of the un-reacted CH₄ to CO and H₂ in the SMR; and (d) reacting the H₂ produced in (c) with N₂ to yield ammonia (NH₃).

In some embodiments of aspects provided herein, the method further comprises separating air to produce oxygen (O₂) and nitrogen (N₂) and directing a first portion of the O₂ into the OCM reactor. In some embodiments of aspects provided herein, the N₂ reacted in (d) comprises at least a portion of the N₂ that was separated from the air. In some embodiments of aspects provided herein, the method further comprises converting a second portion of the un-reacted CH₄ to CO₂ and H₂ in a secondary reformer using a second portion of the O₂. In some embodiments of aspects provided herein, the N₂ reacted in (d) comprises at least a portion of the N₂ that was separated from the air.

An aspect of the present disclosure provides a system for producing ammonia (NH₃) and hydrocarbon compounds containing at least two carbon atoms (C₂₊ compounds), comprising: (a) an oxidative coupling of methane (OCM) module that accepts methane (CH₄) and oxygen (O₂) and reacts the CH₄ and O₂ in an OCM process that yields the C₂₊ compounds, carbon monoxide (CO), hydrogen (H₂), and un-reacted CH₄; and (b) an ammonia production module in fluid communication with the OCM module, wherein the ammonia production module (i) accepts the CO, the H₂, and the un-reacted CH₄ from the OCM module, and (ii) produces NH₃ from the CO, the H₂, and the un-reacted CH₄.

In some embodiments of aspects provided herein, the system further comprises (c) an air separation module in fluid communication with the OCM module and the ammonia production module, wherein the air separation module separates air into an oxygen stream and a nitrogen stream and (i) provides a portion of the oxygen stream to the OCM module, (ii) provides a portion of the oxygen stream to the ammonia production module, and/or (iii) provides the nitrogen stream to the ammonia production module.

An aspect of the present disclosure provides a system for producing ammonia (NH₃) and hydrocarbon compounds containing at least two carbon atoms (C₂+ compounds), comprising an air separation module in fluid communication with an OCM module and an ammonia production module, wherein the air separation module separates air into an oxygen stream and a nitrogen stream and (i) provides a portion of the oxygen stream to the OCM module, (ii) provides a portion of the oxygen stream to the ammonia production module, and/or (iii) provides the nitrogen stream to the ammonia production module.

In some embodiments of aspects provided herein, the oxidative coupling of methane (OCM) module accepts methane (CH₄) and oxygen (O₂) and reacts the CH₄ and O₂ in an OCM process that yields the C₂+ compounds, carbon monoxide (CO), hydrogen (H₂), and un-reacted CH₄. In some embodiments of aspects provided herein, the ammonia production module (i) accepts the CO, the H₂, and the un-reacted CH₄ from the OCM module, and (ii) produces NH₃ from the CO, the H₂, and the un-reacted CH₄.

An aspect of the present disclosure provides a method for producing hydrocarbon compounds containing at least two carbon atoms (C₂+ compounds), comprising: (a) directing a methanol product stream comprising methanol into a methanol-to-propylene (MTP) reactor that reacts the methanol to yield an MTP product stream comprising propylene and hydrocarbon compounds containing at least four carbon atoms (C₄₊compounds); (b) directing the MTP product stream into a separations system that separates the MTP product stream to yield a first stream comprising propylene and a second stream comprising the C₄₊ compounds; (c) directing methane (CH₄) and oxygen (O₂) into an oxidative coupling of methane (OCM) reactor that reacts the CH₄ and the O₂ in an OCM process to yield an OCM product stream comprising the C₂₊ compounds, carbon dioxide (CO₂), hydrogen (H₂), and un-reacted CH₄; and (d) directing the OCM product stream into the separations system, and in the separations system, separating the OCM product stream to yield a third stream comprising ethylene.

In some embodiments of aspects provided herein, the method further comprises, before directing the OCM product stream into the separations system, removing CO₂ from the OCM product stream in a CO₂ removal unit and directing the CO₂ into the methanol reactor. In some embodiments of aspects provided herein, the method further comprises, before directing the OCM product stream into the separations system, removing CH₄ from the OCM product stream in a demethanizer unit and directing the CH₄ into the syngas reactor. In some embodiments of aspects provided herein, the method further comprises, before directing the OCM product stream into the separations system, removing water (H₂O) from the OCM product stream in a drying unit. In some embodiments of aspects provided herein, the method further comprises generating the methanol product stream directing a syngas product stream comprising (i) hydrogen (H₂) and (ii) carbon monoxide (CO) and/or carbon dioxide (CO₂) into a methanol reactor that reacts the H₂ and the CO and/or CO₂ to yield the methanol product stream. In some embodiments of aspects provided herein, the method further comprises generating the syngas product stream by directing a syngas feed stream comprising CH₄ into a syngas reactor that reacts the CH₄ in the syngas feed stream to yield the syngas product stream.

An aspect of the present disclosure provides a system for producing hydrocarbon compounds containing at least two carbon atoms (C₂₊ compounds), comprising: a methanol-to-propylene (MTP) reactor that receives a methanol product stream comprising methanol and reacts the methanol to yield an MTP product stream comprising propylene and hydrocarbon compounds containing at least four carbon atoms (C₄₊ compounds); a separations system in fluid communication with the MTP reactor, wherein the separation system receives the MTP product stream and separates the MTP product stream to yield a first stream comprising propylene and a second stream comprising the C₄₊ compounds; and an oxidative coupling of methane (OCM) reactor that (i) receives methane (CH₄) and oxygen (O₂), (ii) reacts the CH₄ and the O₂ in an OCM process to yield an OCM product stream comprising the C₂₊ compounds, carbon dioxide (CO₂), hydrogen (H₂), and un-reacted CH₄, and (iii) directs the OCM product stream into the separations system to yield a third stream comprising ethylene.

In some embodiments of aspects provided herein, the system further comprises a CO₂ removal unit located downstream of the OCM reactor and upstream of the separations system that removes CO₂ from the OCM product stream and directs the CO₂ into the methanol reactor. In some embodiments of aspects provided herein, the system further comprises a demethanizer unit located downstream of the OCM reactor and upstream of the separations system that removes CH₄ from the OCM product stream directs the CH₄ into the syngas reactor. In some embodiments of aspects provided herein, the system further comprises a drying unit located downstream of the OCM reactor and upstream of the separations system that removes water (H₂O) from the OCM product stream. In some embodiments of aspects provided herein, the system further comprises a methanol reactor that receives a syngas product stream comprising (i) hydrogen (H₂) and (ii) carbon monoxide (CO) and/or carbon dioxide (CO₂) and reacts the H₂ and the CO and/or CO₂ to yield the methanol product stream. In some embodiments of aspects provided herein, the system further comprises a syngas reactor that receives a syngas feed stream comprising CH₄ and reacts the CH₄ in the syngas feed stream to yield the syngas product stream.

An aspect of the present disclosure provides a method for producing liquid natural gas (LNG), comprising: (a) directing methane (CH₄) from a gas processing plant and oxygen (O₂) into an oxidative coupling of methane (OCM) reactor that reacts the CH₄ and the O₂ in an OCM process to yield an OCM product stream comprising hydrocarbon compounds containing at least two carbon atoms (C₂+ compounds); (b) directing the OCM product stream into an ethylene-to-liquids (ETL) reactor that reacts the C₂₊ compounds in an ETL process to yield an ETL product stream comprising compounds containing at least five carbon atoms (C₅₊ compounds); and (c) directing the C₅₊ compounds from the ETL product stream to a liquid petroleum gas (LPG) module of the gas processing plant, which LPG module produces condensate from petroleum gas.

In some embodiments of aspects provided herein, the method further comprises directing C₂ compounds from an LPG extraction unit of the gas processing plant into the OCM reactor. In some embodiments of aspects provided herein, the method further comprises directing the C₅₊ compounds along with condensate from the LPG module. In some embodiments of aspects provided herein, the method further comprises directing at least a portion of the ETL product stream into a gas treatment unit of the gas processing plant.

An aspect of the present disclosure provides a system for producing liquid natural gas (LNG), comprising: an oxidative coupling of methane (OCM) reactor that receives methane (CH₄) from a gas processing plant and oxygen (O₂) and reacts the CH₄ and the O₂ in an OCM process to yield an OCM product stream comprising hydrocarbon compounds containing at least two carbon atoms (C₂+ compounds); and an ethylene-to-liquids (ETL) module in fluid communication with the OCM reactor, wherein the ETL module (i) receives the OCM product stream, (ii) reacts the C₂₊ compounds in an ETL process to yield an ETL product stream comprising compounds containing at least five carbon atoms (C₅₊ compounds), and (iii) directs the C₅₊ compounds from the ETL product stream to a liquid petroleum gas (LPG) module of the gas processing plant, which LPG module produces condensate from petroleum gas.

In some embodiments of aspects provided herein, the system further comprises an LPG extraction unit of the gas processing plant that directs C₂ compounds into the OCM reactor. In some embodiments of aspects provided herein, the ETL modules directs the C₅₊ compounds along with condensate from the LPG module. In some embodiments of aspects provided herein, the ETL modules directs at least a portion of the ETL product stream into a gas treatment unit of the gas processing plant.

An aspect of the present disclosure provides a method for producing polyethylene, comprising: (a) directing C₂ compounds from a liquid petroleum gas (LPG) extraction unit of a gas processing plant into a C₂ splitting unit that separates the C₂ compounds to yield an ethane stream comprising ethane and an ethylene stream comprising ethylene; (b) directing the ethylene stream into a polyethylene reactor that reacts the ethylene in the ethylene stream to yield a polyethylene product stream comprising polyethylene; and (c) directing the ethane stream, methane (CH₄) from the LPG extraction unit, and oxygen (O₂) into an oxidative coupling of methane (OCM) reactor that reacts the CH₄ and the O₂ in an OCM process to yield an OCM product stream comprising hydrocarbon compounds containing at least two carbon atoms (C₂+ compounds).

In some embodiments of aspects provided herein, the method further comprises directing the OCM product stream into a gas treatment unit of the gas processing plant.

An aspect of the present disclosure provides a system for producing polyethylene, comprising: a C₂ splitting unit that receives C₂ compounds from a liquid petroleum gas (LPG) extraction unit of a gas processing plant and separates the C₂ compounds to yield an ethane stream comprising ethane and an ethylene stream comprising ethylene; a polyethylene reactor that receives the ethylene stream and reacts the ethylene in the ethylene stream to yield a polyethylene product stream comprising polyethylene; and an oxidative coupling of methane (OCM) reactor that receives the ethane stream, methane (CH₄) from the LPG extraction unit, and oxygen (O₂) and reacts the CH₄ and the O₂ in an OCM process to yield an OCM product stream comprising hydrocarbon compounds containing at least two carbon atoms (C₂+ compounds).

In some embodiments of aspects provided herein, the system further comprises a gas treatment unit of the gas processing plant that receives the OCM product stream.

An aspect of the present disclosure provides a method for producing oxalate compounds, comprising: (a) directing methane (CH₄) and oxygen (O₂) into an oxidative coupling of methane (OCM) reactor that reacts the CH₄ and the O₂ in an OCM process to yield an OCM product stream comprising hydrocarbon compounds containing at least two carbon atoms (C₂+ compounds) and carbon monoxide (CO) and/or carbon dioxide (CO₂); and (b) directing the CO and/or CO₂ from the OCM product stream into an oxalate reactor that reacts the CO and/or CO₂ to yield an oxalate product stream comprising oxalic acid and/or an oxalate.

In some embodiments of aspects provided herein, the method further comprises directing the oxalate product stream into a hydrogenation reactor that reacts the oxalic acid and/or the oxalate to yield an oxalate derivate product. In some embodiments of aspects provided herein, the oxalate derivative product is selected from the group consisting of glycolic acid, ethylene glycol, diglycolic acid, nitriloacetic acid, glyoxylic acid, acetic acid, salts thereof, and combinations thereof. In some embodiments of aspects provided herein, the oxalate reactor is an electrochemical reactor. In some embodiments of aspects provided herein, the method further comprises directing H₂ from the OCM product stream, from a propane dehydrogenation unit, from a steam reformer, from a water electrolysis unit, from a steam electrolysis unit, or any combination thereof into the oxalate reactor. In some embodiments of aspects provided herein, at least 50% of the CO₂ produced by the OCM reactor is converted into oxalic acid and/or an oxalate.

An aspect of the present disclosure provides a system for producing oxalate compounds, comprising: an oxidative coupling of methane (OCM) reactor that receives methane (CH₄) and oxygen (O₂) and reacts the CH₄ and the O₂ in an OCM process to yield an OCM product stream comprising hydrocarbon compounds containing (i) at least two carbon atoms (C₂+ compounds) and (ii) carbon monoxide (CO) and/or carbon dioxide (CO₂); and an oxalate reactor that receives the CO and/or CO₂ from the OCM product stream and reacts the CO and/or CO₂ to yield an oxalate product stream comprising oxalic acid and/or an oxalate.

In some embodiments of aspects provided herein, the system further comprises a hydrogenation reactor that receives the oxalate product stream and reacts the oxalic acid and/or the oxalate to yield an oxalate derivate product. In some embodiments of aspects provided herein, the oxalate derivative product is selected from the group consisting of glycolic acid, ethylene glycol, diglycolic acid, nitriloacetic acid, glyoxylic acid, acetic acid, salts thereof, and combinations thereof. In some embodiments of aspects provided herein, the oxalate reactor is an electrochemical reactor. In some embodiments of aspects provided herein, the oxalate reactor receives H₂ from the OCM product stream. In some embodiments of aspects provided herein, at least 50% of the CO₂ produced by the OCM reactor is converted into oxalic acid and/or an oxalate.

An aspect of the present disclosure provides a method for producing ethylene derivatives, comprising: (a) directing a methane (CH₄) stream comprising CH₄ and a first oxygen (O₂) stream comprising O₂ into an oxidative coupling of methane (OCM) reactor that reacts the CH₄ and the O₂ in the CH₄ stream and O₂ stream, respectively, in an OCM process to yield an OCM product stream comprising hydrocarbon compounds containing at least two carbon atoms (C₂+ compounds) including ethylene; and (b) directing the ethylene from the OCM product stream and a second O₂ stream comprising O₂ into an oxidation reactor that reacts the ethylene and the O₂ in the second O₂ stream to yield an oxidation product stream comprising ethylene oxide.

In some embodiments of aspects provided herein, the method further comprises directing the oxidation product stream into a hydration reactor that reacts the ethylene oxide to yield ethylene glycol.

An aspect of the present disclosure provides a system for producing ethylene derivatives, comprising: an oxidative coupling of methane (OCM) reactor that receives a methane (CH₄) stream comprising CH₄ and a first oxygen (O₂) stream comprising O₂ and reacts the CH₄ and the O₂ in the CH₄ stream and O₂ stream, respectively, in an OCM process to yield an OCM product stream comprising hydrocarbon compounds containing at least two carbon atoms (C₂+ compounds) including ethylene; and an oxidation reactor that receives the ethylene from the OCM product stream and a second O₂ stream comprising O₂ and reacts the ethylene and the O₂ in the second O₂ stream to yield an oxidation product stream comprising ethylene oxide.

In some embodiments of aspects provided herein, the system further comprises a hydration reactor that receives the oxidation product stream and reacts the ethylene oxide to yield ethylene glycol.

An aspect of the present disclosure provides a method for producing propylene, comprising: (a) directing methane (CH₄) and oxygen (O₂) into an oxidative coupling of methane (OCM) reactor that reacts the CH₄ and the O₂ to yield an OCM product stream comprising hydrocarbon compounds containing at least two carbon atoms (C₂₊ compounds) including ethylene; (b) directing the OCM product stream into a separations unit that yields an ethylene stream comprising ethylene from the OCM product stream; (c) directing a first portion of ethylene from the ethylene stream into a dimerization reactor that reacts the ethylene in a dimerization reaction to yield a butene stream comprising butene compounds; (d) directing the butene stream into a C₄ separations unit that yields a butene-2 stream comprising butene-2 from the butene stream; and (e) directing the butene-2 stream and a second portion of ethylene from the ethylene stream into a metathesis reactor that reacts the butene-2 and the ethylene to yield a metathesis product stream comprising C₂₊ compounds including propylene.

In some embodiments of aspects provided herein, the method further comprises directing the metathesis product stream into a C₂ separations unit that separates the metathesis product stream to yield a C₂ stream comprising C₂ compounds and a C₃₊ stream comprising C₃₊ compounds including propylene. In some embodiments of aspects provided herein, the method further comprises directing the C₂ stream into the separations unit. In some embodiments of aspects provided herein, the method further comprises directing the C₃₊ stream into a C₃ separations unit that separates the C₃₊ stream to yield a C₃ stream comprising propylene and a C₄₊ stream comprising C₄₊ products. In some embodiments of aspects provided herein, the method further comprises directing the C₄₊ stream into the C₄ separations unit. In some embodiments of aspects provided herein, the method further comprises directing the propylene from the metathesis product stream into a polypropylene unit that reacts the propylene to yield a polypropylene product stream comprising polypropylene. In some embodiments of aspects provided herein, the method further comprises directing ethylene from the separations unit to the polypropylene unit, wherein the polypropylene unit reacts the ethylene as a co-monomer with the propylene. In some embodiments of aspects provided herein, the ratio of ethylene co-monomer to total monomer and co-monomer is from about 0.01:0.99 to about 0.15:0.85 In some embodiments of aspects provided herein, the ratio of ethylene co-monomer to total monomer and co-monomer is from about 0.08:0.92 to about 0.15:0.85. In some embodiments of aspects provided herein, step (a) further comprises directing ethane (C₂H₆) into the OCM reactor.

An aspect of the present disclosure provides a system for producing propylene, comprising: an oxidative coupling of methane (OCM) reactor that receives methane (CH₄) and oxygen (O₂) and reacts the CH₄ and the O₂ to yield an OCM product stream comprising hydrocarbon compounds containing at least two carbon atoms (C₂+ compounds) including ethylene; a separations unit that receives the OCM product stream and yields an ethylene stream comprising ethylene from the OCM product stream; a dimerization reactor that receives a first portion of ethylene from the ethylene stream and reacts the ethylene in a dimerization reaction to yield a butene stream comprising butene compounds; a C₄ separations unit that receives the butene stream and yields a butene-2 stream comprising butene-2 from the butene stream; and a metathesis reactor that receives the butene-2 stream and a second portion of ethylene from the ethylene stream and reacts the butene-2 and the ethylene to yield a metathesis product stream comprising C₂₊ compounds including propylene.

In some embodiments of aspects provided herein, the system further comprises a C₂ separations unit that receives the metathesis product stream and separates the metathesis product stream to yield a C₂ stream comprising C₂ compounds and a C₃₊ stream comprising C₃₊ compounds including propylene. In some embodiments of aspects provided herein, the separations unit receives the C₂ stream. In some embodiments of aspects provided herein, the system further comprises a C₃ separations unit that receives the C₃₊ stream and separates the C₃₊ stream to yield a C₃ stream comprising propylene and a C₄₊ stream comprising C₄₊ products. In some embodiments of aspects provided herein, the C₄ separations unit receives the C₄₊ stream. In some embodiments of aspects provided herein, the system further comprises a polypropylene unit that receives the propylene from the metathesis product stream and reacts the propylene to yield a polypropylene product stream comprising polypropylene. In some embodiments of aspects provided herein, the polypropylene unit receives ethylene from the separations unit and reacts the ethylene as a co-monomer with the propylene. In some embodiments of aspects provided herein, the ratio of ethylene co-monomer to total monomer and co-monomer is from about 0.01:0.99 to about 0.15:0.85 In some embodiments of aspects provided herein, the ratio of ethylene co-monomer to total monomer and co-monomer is from about 0.08:0.92 to about 0.15:0.85. In some embodiments of aspects provided herein, the OCM reactor receives ethane (C₂H₆).

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF THE FIGURES

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings or figures (also referred to herein as "FIG." and "FIGs."), of which:
**FIG. 1A** is a schematic illustration of an oxidative coupling of methane (OCM) process;
**FIG. 1B** is a schematic illustration of an oxidative coupling of methane (OCM) process with separate addition of ethane;
**FIG. 1C** shows a block flow diagram of an OCM process that includes ethane conversion, separations and methanation;
**FIG. 1D** shows a process block flow diagram with feeds and products;
**FIG. 1E** shows a process block flow diagram with carbon utilization;
**FIG. 2** is a schematic illustration of addition of ethane to an OCM reactor;
**FIG. 3** is a schematic illustration of a methanol production process;
**FIG. 4** is a schematic illustration of OCM integrated with a methanol production process;
**FIG. 5** is a schematic illustration of a petrochemical complex with a methanol production process and a cracker;
**FIG. 6** is a schematic illustration of the integration of OCM with a methanol production process and a cracker;
**FIG. 7** is a schematic illustration of the integration of OCM with a methanol production process and a cracker;
**FIG. 8** is a schematic illustration of the integration of OCM with a methanol production process and a cracker;
**FIG. 9** is a schematic illustration of a chloralkali process;
**FIG. 10** is a schematic illustration of OCM integrated with a chloralkali process;
**FIG. 11** is a schematic illustration of an ethylene dichloride (EDC) and vinylchloride monomer (VCM) production process;
**FIG. 12** is a schematic illustration of an EDC/VCM process integrated with a chloralkali process;
**FIG. 13** is a schematic illustration of OCM integrated with an EDC/VCM process and a chloralkali process;
**FIG. 14** shows an example of a process integrating OCM with a diaphragm-type chloralkali process;
**FIG. 15** shows a material and energy balance for the process shown in **FIG. 14****;**
**FIG. 16** is a schematic illustration of OCM integrated with an ammonia process;
**FIG. 17** shows a schematic illustration of OCM integrated with a methanol-to-propylene (MTP) process.
**FIG. 18** shows a schematic illustration of an OCM process integrated with an MTP process.
**FIG. 19** shows a schematic illustration of an OCM process and an ETL process integrated with a liquid natural gas (LNG) process.
**FIG. 20** shows a schematic illustration of OCM and ETL processes integrated with an LNG process for polymer production.
**FIG. 21** shows a schematic illustration of an OCM process integrated with an oxalic acid/oxalate production process.
**FIG. 22** shows a schematic illustration of an OCM process integrated with an ethylene glycol production process.
**FIG. 23** shows a schematic illustration of an OCM process integrated with a metathesis-based propylene production process.
**FIG. 24** shows a schematic illustration of an OCM process integrated with a metathesis-based propylene production process with polypropylene production.
**FIG. 25A** shows a schematic illustration of an OCM process integrated with a metathesis-based propylene production process having a C₂ splitter.
**FIG. 25B** shows a shows a schematic illustration of an OCM process integrated with a metathesis-based propylene production process without a C₂ splitter.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

The term "higher hydrocarbon," as used herein, generally refers to a higher molecular weight and/or higher chain hydrocarbon. A higher hydrocarbon can have a higher molecular weight and/or carbon content that is higher or larger relative to starting material in a given process (e.g., OCM or ETL). A higher hydrocarbon can be a higher molecular weight and/or chain hydrocarbon product that is generated in an OCM or ETL process. For example, ethylene is a higher hydrocarbon product relative to methane in an OCM process. As another example, a C₃₊ hydrocarbon is a higher hydrocarbon relative to ethylene in an ETL process. As another example, a C₅₊ hydrocarbon is a higher hydrocarbon relative to ethylene in an ETL process. In some cases, a higher hydrocarbon is a higher molecular weight hydrocarbon.

The term "OCM process," as used herein, generally refers to a process that employs or substantially employs an oxidative coupling of methane (OCM) reaction. An OCM reaction can include the oxidation of methane to a higher hydrocarbon and water, and involves an exothermic reaction. In an OCM reaction, methane can be partially oxidized and coupled to form one or more C₂₊ compounds, such as ethylene. In an example, an OCM reaction is 2CH₄ + O₂ → C₂H₄ + 2H₂O. An OCM reaction can yield C₂₊ compounds. An OCM reaction can be facilitated by a catalyst, such as a heterogeneous catalyst. Additional by-products of OCM reactions can include CO, CO₂, H₂, as well as hydrocarbons, such as, for example, ethane, propane, propene, butane, butene.

The term "non-OCM process," as used herein, generally refers to a process that does not employ or substantially employ an oxidative coupling of methane reaction. Examples of processes that may be non-OCM processes include non-OCM hydrocarbon processes, such as, for example, non-OCM processes employed in hydrocarbon processing in oil refineries, a natural gas liquids separations processes, steam cracking of ethane, steam cracking or naphtha, Fischer-Tropsch processes.

The terms "C₂₊" and "C₂+ compound," as used herein, generally refer to a compound comprising two or more carbon atoms. For example, C₂₊ compounds include, without limitation, alkanes, alkenes, alkynes and aromatics containing two or more carbon atoms. C₂₊ compounds can include aldehydes, ketones, esters and carboxylic acids. Examples of C₂₊ compounds include ethane, ethene, acetylene, propane, propene, butane, and butene.

The term "non-C₂₊ impurities," as used herein, generally refers to material that does not include C₂₊ compounds. Examples of non-C₂₊ impurities, which may be found in certain OCM reaction product streams, include nitrogen (N₂), oxygen (O₂), water (H₂O), argon (Ar), hydrogen (H₂) carbon monoxide (CO), carbon dioxide (CO₂) and methane (CH₄).

The term "small scale," as used herein, generally refers to a system that generates less than or equal to about 250 kilotons per annum (KTA) of a given product, such as an olefin (e.g., ethylene).

The term "world scale," as used herein, generally refers to a system that generates greater than about 250 KTA of a given product, such as an olefin (e.g., ethylene). In some examples, a world scale olefin system generates at least about 1000, 1100, 1200, 1300, 1400, 1500, or 1600 KTA of an olefin.

The term "item of value," as used herein, generally refers to money, credit, a good or commodity (e.g., hydrocarbon). An item of value can be traded for another item of value.

The term "carbon efficiency," as used herein, generally refers to the ratio of the number of moles of carbon present in all process input streams (in some cases including all hydrocarbon feedstocks, such as, e.g., natural gas and ethane and fuel streams) to the number of moles of carbon present in all commercially (or industrially) usable or marketable products of the process. Such products can include hydrocarbons that can be employed for various downstream uses, such as petrochemical or for use as commodity chemicals. Such products can exclude CO and CO₂. The products of the process can be marketable products, such as C₂₊ hydrocarbon products containing at least about 99% C₂₊ hydrocarbons and all sales gas or pipeline gas products containing at least about 90% methane. Process input streams can include input streams providing power for the operation of the process, such as with the aid of a turbine (e.g., steam turbine). In some cases, power for the operation of the process can be provided by heat liberated by an OCM reaction.

The term "nitrogen efficiency," as used herein, generally refers to the ratio of the number of moles of nitrogen present in all process input streams (in some cases including all nitrogen feedstocks, such as, e.g., air or purified nitrogen) to the number of moles of nitrogen present in all commercially (or industrially) usable or marketable products of the process. Such products can include ammonia and other nitrogen products that can be employed for various downstream uses, such as petrochemical use, agricultural use, or for use as commodity chemicals. Such products can exclude nitrogen oxides (NOx), such as NO and NO₂. The products of the process can be marketable products, such as ammonia and derivatives thereof containing at least about 90% or 99% ammonia or ammonia derivatives. Process input streams can include input streams providing power for the operation of the process, such as with the aid of a turbine (e.g., steam turbine). In some cases, power for the operation of the process can be provided by heat liberated by a reaction, such as an OCM reaction.

The term "C₂+ selectivity," as used herein, generally refers to the percentage of the moles of methane that are converted into C₂₊ compounds.

The term "specific oxygen consumption," as used herein, generally refers to the mass (or weight) of oxygen consumed by a process divided by the mass of C₂₊ compounds produced by the process.

The term "specific CO₂ emission," as used herein, generally refers to the mass of CO₂ emitted from the process divided by the mass of C₂₊ compounds produced by the process.

### OCM Processes

In an OCM process, methane (CH₄) reacts with an oxidizing agent over a catalyst bed to generate C₂₊ compounds. For example, methane can react with oxygen over a suitable catalyst to generate ethylene, e.g., 2 CH₄ + O₂ → C₂H₄ + 2 H₂O (*See, e.g.,* Zhang, Q., Journal of Natural Gas Chem., 12:81, 2003; Olah, G. "Hydrocarbon Chemistry", Ed. 2, John Wiley & Sons (2003)). This reaction is exothermic (ΔH = -280 kJ/mol) and has typically been shown to occur at very high temperatures (e.g., >450°C or >700°C). Non-selective reactions that can occur include (a) CH₄ + 2O₂ → CO₂ + 2 H₂O and (b) CH₄ + 1/2 O₂ → CO + 2 H₂. These non-selective reactions are also exothermic, with reaction heats of -891 kJ/mol and -36 kJ/mol respectively. The conversion of methane to COx products is undesirable due to both heat management and carbon efficiency concerns.

Experimental evidence suggests that free radical chemistry is involved. (Lunsford, J. Chem. Soc., Chem. Comm., 1991; H. Lunsford, Angew. Chem., Int. Ed. Engl., 34:970, 1995). In the reaction, methane (CH₄) is activated on the catalyst surface, forming methyl radicals which then couple on the surface or in the gas phase to form ethane (C₂H₆), followed by dehydrogenation to ethylene (C₂H₄). The OCM reaction pathway can have a heterogeneous/ homogeneous mechanism, which involves free radical chemistry. Experimental evidence has shown that an oxygen active site on the catalyst activates the methane, removes a single hydrogen atom and creates a methyl radical. Methyl radicals react in the gas phase to produce ethane, which is either oxidative or non-oxidatively dehydrogenated to ethylene. The main reactions in this pathway can be as follows: (a) CH₄ + O⁻ → CH₃^{∗} + OH⁻; (b) 2 CH₃^{∗}→ C₂H₆; (c) C₂H₆ + O⁻ → C₂H₄ + H₂O. In some cases, to improve the reaction yield, ethane can be introduced downstream of the OCM catalyst bed and thermally dehydrogenated via the following reaction: C₂H₆ → C₂H₄ + H₂. This reaction is endothermic (ΔH = 144 kJ/mol), which can utilize the exothermic reaction heat produced during methane conversion. Combining these two reactions in one vessel can increase thermal efficiency while simplifying the process.

Several catalysts have shown activity for OCM, including various forms of iron oxide, V₂O₅, MoO₃, Co₃O₄, Pt-Rh, Li/ZrO₂, Ag-Au, Au/Co₃O₄, Co/Mn, CeO₂, MgO, La₂O₃, Mn₃O₄, Na₂WO₄, MnO, ZnO, and combinations thereof, on various supports. A number of doping elements have also proven to be useful in combination with the above catalysts.

Since the OCM reaction was first reported over thirty years ago, it has been the target of intense scientific and commercial interest, but the fundamental limitations of the conventional approach to C-H bond activation appear to limit the yield of this attractive reaction under practical operating conditions. Specifically, numerous publications from industrial and academic labs have consistently demonstrated characteristic performance of high selectivity at low conversion of methane, or low selectivity at high conversion (J.A. Labinger, Cat. Lett., 1:371, 1988). Limited by this conversion/selectivity threshold, no OCM catalyst has been able to exceed 20-25% combined C₂ yield (i.e., ethane and ethylene), and more importantly, all such reported yields required extremely high reactor inlet temperatures (> 800°C). Novel catalysts and processes have been described for use in performing OCM in the production of ethylene from methane at substantially more practicable temperatures, pressures and catalyst activities. These are described in U.S. Patent Application Serial Nos. 13/115,082, 13/479,767, 13/689,611, 13/689,514, 13/901,319, 14/212,435, and 14/701,963.

An OCM reactor can include a catalyst that facilitates an OCM process. The catalyst may include a compound including at least one of an alkali metal, an alkaline earth metal, a transition metal, and a rare-earth metal. The catalyst may be in the form of a honeycomb, packed bed, or fluidized bed. In some embodiments, at least a portion of the OCM catalyst in at least a portion of the OCM reactor can include one or more OCM catalysts and/or nanostructure-based OCM catalyst compositions, forms and formulations. Examples of OCM reactors, separations for OCM, and OCM process designs are described in U.S. Patent Application Serial Nos. 13/739,954, 13/900,898, 13/936,783, 14/553,795, and 14/592,688. An OCM reactor can be adiabatic or substantially adiabatic (including, for example, a post-bed cracking unit). An OCM reactor can be isothermal or substantially isothermal.

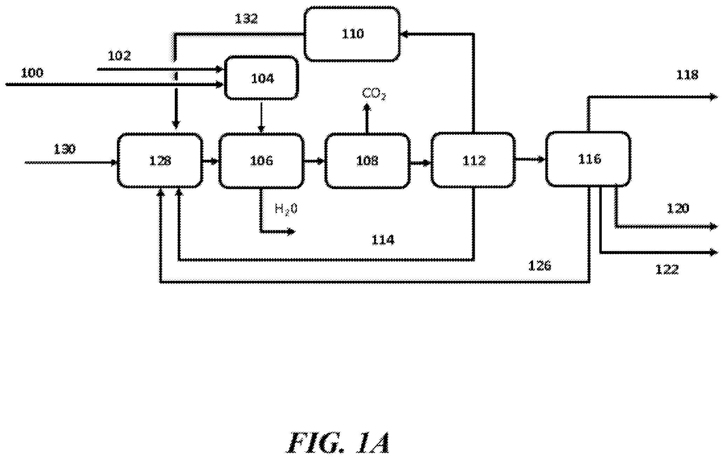

With reference to **FIG. 1A****,** natural gas **100** and ethane **102** can enter the process through a de-sulfurization module **104,** which can flow into a process gas compression module **106** where water can be removed. OCM product gas can be added to the process gas compression module **106** as well. A process gas cleanup module **108** can remove carbon dioxide (CO₂), some or all which can be taken to a methanation module **110.** Following cleanup, the process gas can flow into a first separations module **112** that removes C₂₊ compounds from the process gas stream. The remaining process gas can flow to the methanation module **110** and/or a fired heater (e.g., to heat incoming OCM gas streams **114).** The C₂₊ compounds can be fractionated in a second separations module **116** to produce ethylene (C₂H₄) **118,** C₃ compounds **120,** and C₄₊ compounds **122** for example. The second separations module **116** can produce an ethane (C2H6) stream **126** that is returned to the OCM reactor **128.** At the OCM reactor **128,** oxygen **130** can be reacted with methane from the methanation module **132.** Outside boundary limits (OSBL) systems include a steam system, a boiler feed water system and a cooling water system.

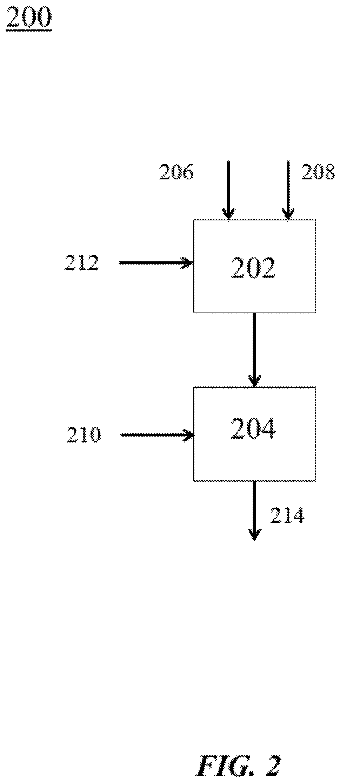

The OCM reactor can perform the OCM reaction and post-bed cracking (PBC), as described in U.S. Patent Application Serial No. 14/553,795. With reference to **FIG. 2****,** the OCM reactor **200** can have an OCM reaction section **202** and a PBC section **204.** Methane **206** (e.g., natural gas) and oxygen **208** can be injected (via a mixer) in to the OCM reaction region (which comprises an OCM catalyst). The OCM reaction is exothermic and the heat of reaction can be used to crack additional ethane **210** that can be injected into the PBC region **204.** In some cases, yet more ethane **212** is also injected into the OCM reaction region **202** and/or the methane feed is supplemented with ethane or other C₂₊ alkanes (e.g., propane or butane). The OCM reactor produces an OCM effluent **214.**

The relative amounts of supplemental ethane **210** and **212** can be varied to achieve a range of product outcomes from the system. In some cases, no ethane is injected into the OCM reaction region **202** (referred to herein as Case-1). Another case presented herein has 3.5 mol% ethane injected into the OCM region (referred to herein as Case-2). Some process design results are presented in **Table 1.**

**Table 1: Examples of various amounts of ethane in OCM feed**

| | **Case-1** | **Case-2** |
|---|---|---|
| Natural gas consumed (MMSCFD) | 15.5 | 16 |
| Ethane consumed (MMSCFD) | 2.2 | 8.3 |
| [Ethane] at inlet (mol%) | 0.07 | 3.5 |
| [Ethylene] at outlet (mol%) | 3.6 | 4.9 |
| C₂ products (kTa) | 85 | 115 |
| C₃ products (kTa) | 10.3 | 21.1 |
| C₄₊ products (kTa) | 2.7 | 2.5 |
| O₂ consumed (ton/ton ethylene) | 2.2 | 1.8 |
| CO₂ produced from OCM (ton/ton ethylene) | 0.9 | 0.7 |
| CO₂ produced from fired heater (ton/ton ethylene) | 0.6 | 0.4 |

In some cases, the amount of hydrogen (H₂) exiting the OCM reactor is relatively higher for cases having relatively more ethane injection (e.g., 8% H₂ for Case-1 and about H₂ 10% for Case-2). The amount of ethane that can be injected can be limited by the desired temperature exiting the OCM reaction region **202** or the OCM reactor **214.**

In some cases, the process equipment is sized to accommodate a range of amounts of additional ethane such that the process is flexible. For example, more ethane can be injected into the process when the price of ethane is relatively cheap in comparison to the price of natural gas (e.g., low frac spread).

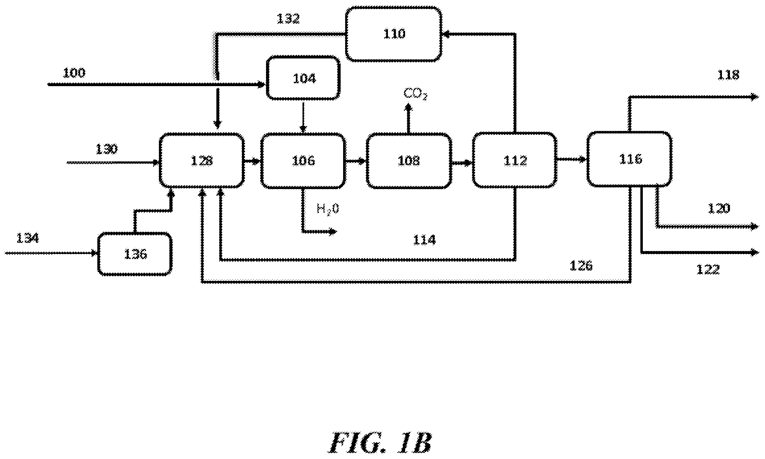

The ethane can be mixed with the natural gas and recycled to the OCM unit (as shown in **FIG. 1A****).** In some cases, with reference to **FIG. 1B****,** the ethane **134** can go straight to the OCM reactor, optionally through a separate de-sulfurization module **136.** Injection of ethane through a separate de-sulfurization module can reduce the load in the recycle loop of the process and/or give additional production capacity keeping the same recirculation rate. The purge gas from the process can be used for fuel gas to the fired heater or sales gas.

The concentration of ethane in the feed to the OCM reactor can be any suitable value, including about 0.0 mol%, about 0.25 mol%, about 0.5 mol%, about 0.75 mol%, about 1.0 mol%, about 1.25 mol%, about 1.5 mol%, about 1.75 mol%, about 2.0 mol%, about 2.25 mol%, about 2.5 mol%, about 2.75 mol%, about 3.0 mol%, about 3.25 mol%, about 3.5 mol%, about 3.75 mol%, about 4.0 mol%, about 4.25 mol%, about 4.5 mol%, about 4.75 mol%, about 5.0 mol%, about 5.25 mol%, about 5.5 mol%, about 5.75 mol%, about 6.0 mol%, or more. In some cases, the concentration of ethane in the feed to the OCM reactor is at least about 0.0 mol%, at least about 0.25 mol%, at least about 0.5 mol%, at least about 0.75 mol%, at least about 1.0 mol%, at least about 1.25 mol%, at least about 1.5 mol%, at least about 1.75 mol%, at least about 2.0 mol%, at least about 2.25 mol%, at least about 2.5 mol%, at least about 2.75 mol%, at least about 3.0 mol%, at least about 3.25 mol%, at least about 3.5 mol%, at least about 3.75 mol%, at least about 4.0 mol%, at least about 4.25 mol%, at least about 4.5 mol%, at least about 4.75 mol%, at least about 5.0 mol%, at least about 5.25 mol%, at least about 5.5 mol%, at least about 5.75 mol%, at least about 6.0 mol%, or more. In some cases, the concentration of ethane in the feed to the OCM reactor is at most about 0.0 mol%, at most about 0.25 mol%, at most about 0.5 mol%, at most about 0.75 mol%, at most about 1.0 mol%, at most about 1.25 mol%, at most about 1.5 mol%, at most about 1.75 mol%, at most about 2.0 mol%, at most about 2.25 mol%, at most about 2.5 mol%, at most about 2.75 mol%, at most about 3.0 mol%, at most about 3.25 mol%, at most about 3.5 mol%, at most about 3.75 mol%, at most about 4.0 mol%, at most about 4.25 mol%, at most about 4.5 mol%, at most about 4.75 mol%, at most about 5.0 mol%, at most about 5.25 mol%, at most about 5.5 mol%, at most about 5.75 mol%, or at most about 6.0 mol%.

The systems and methods of the present disclosure can be carbon-efficient and/or energy-efficient. In some cases, the systems or methods of the present disclosure have a carbon efficiency of at least about 50%, at least about 55%, at least about 60%, at least about 65%, at least about 70%, at least about 75%, at least about 80%, at least about 85%, or at least about 90%. In some cases, a system of the present disclosure or method for use thereof has a ratio of all carbon atoms output from the system as hydrocarbons to all carbon atoms input to the system of at least about 0.4, at least about 0.50, at least about 0.55, at least about 0.60, at least about 0.65, at least about 0.70, at least about 0.75, at least about 0.80, at least about 0.85, at least about 0.90, or at least about 0.95.

In some cases, the systems or methods of the present disclosure have a carbon efficiency of between about 50% and about 85%, between about 55% and about 80%, between about 60% and about 80%, between about 65% and about 85%, between about 65% and about 80%, or between about 70% and about 80%. In some cases, a system of the present disclosure or method for use thereof has a ratio of all carbon atoms output from the system as hydrocarbons to all carbon atoms input to the system of between about 0.50 and about 0.85, between about 0.55 and about 0.80, between about 0.60 and about 0.80, between about 0.65 and about 0.85, between about 0.65 and about 0.80, or between about 0.70 and about 0.80.

In some instances, the carbon efficiency is at least about 50%, at least about 55%, at least about 60%, at least about 65%, at least about 70%, at least about 75%, at least about 80%, at least about 85% or at least about 90%. In some instances, the carbon efficiency is between about 50% and about 85%, between about 55% and about 80%, between about 60% and about 80%, between about 65% and about 85%, between about 65% and about 80%, or between about 70% and about 80%. In some instances, a system of the present disclosure or method for use thereof has a ratio of all carbon atoms output from the system as hydrocarbons to all carbon atoms input to the system of at least about 0.60, at least about 0.65, at least about 0.70, at least about 0.75, at least about 0.80, at least about 0.85 or at least about 0.90. In some instances, a system of the present disclosure or method for use thereof has a ratio of all carbon atoms output from the system as hydrocarbons to all carbon atoms input to the system of between about 0.50 and about 0.85, between about 0.55 and about 0.80, between about 0.60 and about 0.80, between about 0.65 and about 0.85, between about 0.65 and about 0.80, or between about 0.70 and about 0.80.

In some cases, the systems and methods combine OCM reaction, post-bed cracking (PBC), separations and methanation. The separations can include oligomerization of ethylene to C₃₊ compounds, which are more easily separated as described in PCT Patent Application No. PCT/US2015/010525. Additional details of OCM reactor and process design can be found in PCT Patent Application No. PCT/US2014/057465 and PCT Patent Application No. PCT/US2015/010688.

In an aspect, provided herein is a method for performing oxidative coupling of methane (OCM). The method can comprise (a) reacting oxygen (O₂) with methane (CH₄) to form heat, ethylene (C₂H₄) and optionally ethane (C₂H₆), hydrogen (H₂), carbon monoxide (CO) or carbon dioxide (CO₂); (b) reacting the heat produced in (a) with ethane (C₂H₆) to form ethylene (C₂H₄) and hydrogen (H₂); (c) performing at least one of (i) enriching the ethylene (C₂H₄) produced in (a) and (b) or (ii) oligomerizing the ethylene (C₂H₄) produced in (a) and (b) to produce C₃₊ compounds and enriching the C₃₊ compounds; and (d) reacting the hydrogen (H₂) produced in (a) and (b) with carbon monoxide (CO) and/or carbon dioxide (CO₂) to form methane (CH₄).

In another aspect, provided herein is a system for performing oxidative coupling of methane (OCM). The system comprises an OCM reactor that reacts oxygen (O₂) with methane (CH₄) to form heat, ethylene (C₂H₄) and optionally ethane (C₂H₆), hydrogen (H₂), carbon monoxide (CO) or carbon dioxide (CO₂). The system further comprises a cracking vessel in fluid communication with the OCM reactor, which cracking vessel reacts the heat produced in the OCM reactor with ethane (C₂H₆) to form ethylene (C₂H₄) and hydrogen (H₂). The system further comprises a separations module in fluid communication with the cracking vessel, which separation module (i) enriches the ethylene (C₂H₄) produced in the OCM reactor and the cracking vessel or (ii) oligomerizes the ethylene (C₂H₄) produced in the OCM reactor and the cracking vessel to produce C₃₊ compounds and enriches the C₃₊ compounds. The system further comprises a methanation reactor in fluid communication with the separations module, which methanation reactor reacts the hydrogen (H₂) produced in the OCM reactor and the cracking vessel with carbon monoxide (CO) and/or carbon dioxide (CO₂) to form methane (CH₄).

In some cases, the ethane (C₂H₆) that is cracked in the cracking vessel was produced in the OCM reactor. In some instances, at least some of the ethane (C₂H₆) that is cracked is in addition to the ethane (C₂H₆) that was produced in the OCM reactor. In some cases, the OCM reactor produces ethane (C₂H₆), hydrogen (H₂), carbon monoxide (CO) and carbon dioxide (CO₂). In some cases, the carbon monoxide (CO) and carbon dioxide (CO₂) produced in the OCM reactor is methanated. The separations module can separate ethylene (C₂H₄) or C₃₊ compounds from methane (CH₄), ethane (C₂H₆), hydrogen (H₂), carbon monoxide (CO) or carbon dioxide (CO₂). In some instances, the cracking vessel is a portion of the OCM reactor.

The methane formed in the methanation reactor can be returned to the OCM reactor or sold as sales gas. In some embodiments, the OCM reactor has an OCM catalyst. In some embodiments, the methanation reactor has a methanation catalyst. In some embodiments, the separations module comprises an ethylene-to-liquids (ETL) reactor comprising an oligomerization catalyst. At least some of the heat produced in the OCM reactor can be converted to power.

In another aspect, described herein is a method for producing C₂₊ compounds from methane (CH₄). The method can comprise: (a) performing an oxidative coupling of methane (OCM) reaction which converts methane (CH₄) and oxygen (O₂) into ethylene (C₂H₄) and optionally ethane (C₂H₆); (b) optionally oligomerizing the ethylene (C₂H₄) to produce C₃₊ compounds; and (c) isolating the C₂₊ compounds, wherein the C₂₊ compounds comprise the ethylene (C₂H₄), the ethane (C₂H₆) and/or the C₃₊ compounds, where the method has a carbon efficiency of at least about 50%. In some cases, the isolated the C₂₊ compounds are not pure. In some cases, the isolated the C₂₊ compounds comprise methane, CO, H₂, CO₂ and/or water.

In some cases, the systems or methods of the present disclosure consume less than about 150, less than about 140, less than about 130, less than about 120, less than about 110, less than about 100, less than about 95, less than about 90, less than about 85, less than about 80, less than about 75, less than about 70, less than about 65, less than about 60, less than about 55, or less than about 50 million British Thermal Units (MMBtu) of energy per ton of ethylene (C₂H₄) or C₃₊ compounds enriched. In some cases, the amount of energy consumed by the system includes the energy content of the feedstock used to make the ethylene (C₂H₄) or C₃₊ compounds.

In some cases, the systems or methods of the present disclosure have consume between about 65 and about 100, between about 70 and about 110, between about 75 and about 120, between about 85 and about 130, between about 40 and about 80, or between about 50 and about 80 MMBtu of energy per ton of ethylene (C₂H₄) or C₃₊ compounds enriched. In some cases, the amount of energy consumed by the system includes the energy content of the feedstock used to make the ethylene (C₂H₄) or C₃₊ compounds.

In some embodiments, the systems or methods of the present disclosure have a specific oxygen consumption of about 1.2, about 1.3, about 1.4, about 1.5, about 1.6, about 1.7, about 1.8, about 1.9, about 2.0, about 2.1, about 2.2, about 2.3, about 2.4, about 2.5, about 2.6 about 2.7, about 2.8, about 2.9, about 3, about 3.2, about 3.4, about 3.6, about 3.8, or about 4.0.

In some embodiments, the systems or methods of the present disclosure have a specific oxygen consumption of between about 1.2 and about 2.7, between about 1.5 and about 2.5, between about 1.7 and about 2.3 or between about 1.9 and about 2.1.

In some embodiments, the systems or methods of the present disclosure have a specific CO₂ emission of about 0.5, about 0.6, about 0.7, about 0.8, about 0.9, about 1.0, about 1.1, about 1.2, about 1.3, about 1.4, about 1.5, about 1.6, about 2.0, about 2.2, about 2.4, about 2.6, about 2.8, about 3.0, about 3.2, about 3.4, or about 3.6.

In some embodiments, the systems or methods of the present disclosure have a specific CO₂ emission of between about 0.5 and about 1.7, between about 0.7 and about 1.4, between about 0.8 and about 1.3 or between about 0.9 and about 1.1.

In some embodiments, the systems or methods of the present disclosure produces C₂₊ products, and the C₂₊ products comprise at least about 2.5%, at least about 2.5%, at least about 5%, at least about 7.5%, at least about 10%, at least about 12.5% or at least about 15% C₃₊ hydrocarbons.

In some embodiments, the systems or methods of the present disclosure produces C₂ products and C₃₊ products, and the ratio of the C₂ products to the C₃₊ products is about 20, about 15, about 10, about 8, about 6 or about 5.

In some embodiments, the systems or methods of the present disclosure produces C₂ products and C₃₊ products, and the ratio of the C₂ products to the C₃₊ products is between about 5 and about 20, between about 6 and about 10, or between about 8 and about 10.

In another aspect, provided herein is a method for producing C₂₊ compounds from methane (CH₄), the method comprising: (a) performing an oxidative coupling of methane (OCM) reaction which converts methane (CH₄) and oxygen (O₂) into ethylene (C₂H₄) and optionally ethane (C₂H₆); (b) optionally oligomerizing the ethylene (C₂H₆) to produce C₃₊ compounds; and (c) isolating the C₂₊ compounds, wherein the C₂₊ compounds comprise the ethylene (C₂H₄), the ethane (C₂H₆) and/or the C₃₊ compounds, where the method consumes less than about 100 MMBtu of energy per ton of the C₂₊ compounds isolated. In some cases, the amount of energy consumed by the system includes the energy content of the feedstock used to make the isolated C₂₊ compounds. In some cases, the isolated the C₂₊ compounds are not pure. In some cases, the isolated the C₂₊ compounds comprise methane, CO, H₂, CO₂ and/or water.

In some cases, the method consumes less than about 150, less than about 140, less than about 130, less than about 120, less than about 110, less than about 100, less than about 95, less than about 90, less than about 85, less than about 80, less than about 75, less than about 70, less than about 65, less than about 60, less than about 55, or less than about 50 MMBtu of energy per ton of C₂₊ compounds isolated. In some cases, the method consumes between about 65 and about 100, between about 70 and about 110, between about 75 and about 120, between about 85 and about 130, between about 40 and about 80, or between about 50 and about 80 MMBtu of energy per ton of C₂₊ compounds isolated.

In another aspect, provided herein is a method for producing C₂₊ compounds from methane (CH₄), the method comprising performing an oxidative coupling of methane (OCM) reaction using an OCM catalyst at a set of reaction conditions to convert a quantity of methane (CH₄) into ethylene (C₂H₄) at a carbon efficiency, where the OCM catalyst has a C₂₊ selectivity at the set of reaction conditions that is less than the carbon efficiency at the set of reaction conditions. The set of reaction conditions can include a temperature, a pressure, a methane to oxygen ratio and a gas hourly space velocity (GHSV).

In another aspect, provided herein is a method for producing C₂₊ compounds from methane (CH₄), the method comprising: (a) performing an oxidative coupling of methane (OCM) reaction using an OCM catalyst at a set of reaction conditions to convert a quantity of methane (CH₄) into ethylene (C₂H₄) and ethane (C₂H₆); and (b) cracking the ethane (C₂H₆) to produce additional ethylene (C₂H₄), where the combined carbon efficiency of (a) and (b) is greater than the C₂₊ selectivity of the OCM catalyst at the set of reaction conditions. The set of reaction conditions can include a temperature, a pressure, a methane to oxygen ratio and a gas hourly space velocity (GHSV).

In some instances, the C₂₊ selectivity is at most about 70%, at most about 65%, at most about 60%, at most about 55%, at most about 50%, at most about 45%, at most about 40%, or at most about 35%. In some instances, the C₂₊ selectivity is at least about 70%, at least about 65%, at least about 60%, at least about 55%, at least about 50%, at least about 45%, at least about 40%, or at least about 35%.

In another aspect, provided herein is a method for producing C₂₊ compounds, the method comprising: (a) providing a first feedstock comprising methane (CH₄) and optionally a first amount of ethane (C₂H₆); (b) performing an OCM reaction on the first feedstock to produce an OCM product comprising a first amount of ethylene (C₂H₄); (c) combining the OCM product with a second feedstock comprising a second amount of ethane (C₂H₆) to produce a third feedstock; and (d) cracking the third feedstock to produce a second amount of ethylene (C₂H₄), where the second amount of ethylene includes ethylene produced in (b) and (d).

In some cases, the fraction of the second amount of ethylene (C₂H₄) that is derived from the first or the second amounts of ethane (C₂H₆) is at least about 1%, at least about 3%, at least about 5%, at least about 7%, at least about 10%, at least about 15%, at least about 20%, at least about 25%, at least about 30%, at least about 35%, at least about 40%, at least about 45%, at least about 50%, or at least about 55%.

In some cases, the combined moles of the first amount and second amount of ethane (C₂H₆) divided by the combined moles of the first feedstock and the second feedstock is about 1%, about 3%, about 5%, about 7%, about 10%, about 15%, about 20%, about 25%, about 30%, about 35%, about 40%, about 45%, about 50%, about 55%, or about 60%.

In some cases, the combined moles of the first amount and second amount of ethane (C₂H₆) divided by the combined moles of the first feedstock and the second feedstock is between about 1% and about 50%, between about 1% and about 40%, between about 1% and about 30%, between about 1% and about 20%, between about 1% and about 15%, between about 1% and about 10%, or between about 10% and about 50%.

In some cases, the first feedstock is natural gas. In some cases, the first feedstock is natural gas supplemented with the first amount of ethane (C₂H₆). In some cases, the first feedstock is natural gas having passed through a separations system to substantially remove the hydrocarbons other than methane.

In some cases, the molar percent of ethane (C₂H₆) in methane (CH₄) in the first feedstock is about 1%, about 3%, about 5%, about 7%, about 10%, about 15% or about 20%.

In some cases, some or all of a methane-containing feed stream (e.g., natural gas) can be processed in a separation system prior to being directed into an OCM reactor. Directing a methane-containing feed stream into an OCM reactor via a separation system or subsystem rather than into an OCM reactor directly can provide advantages, including but not limited to increasing the carbon efficiency of the process, optimizing the OCM process for methane processing, and optimizing the post-bed cracking (PBC) process for ethane processing. Such a configuration can result in higher back-end sizing for the system; however, in some cases (e.g., when using high pressure pipeline natural gas as a feedstock, high recycle ratio), the back-end sizing increase can be reduced or moderated. The separation system or subsystem can comprise a variety of operations including any discussed in the present disclosure, such as CO₂ removal via an amine system, caustic wash, dryers, demethanizers, deethanizers, and C₂ splitters. In some cases, all of the methane and ethane in the methane-containing feed stream (e.g., natural gas) passes through a separations system or separations subsystem prior to passing through an OCM reactor. Some or all of the ethane from the feed stream can be directed from the separation system or subsystem into the inlet of an OCM reactor or into a post-bed cracking (PBC) unit.

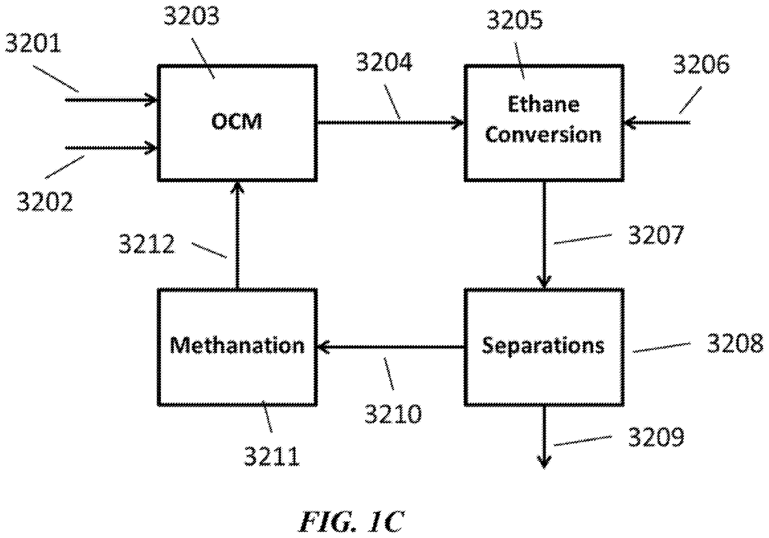

In some configurations, an OCM system can be operated in a cycle, with at least some of the products from one unit or subsystem being processed or reacted in the next unit or subsystem (see, e.g., **FIG. 1C**). For example, oxygen (O₂) **3201** and methane (CH₄) feed **3202** can be provided to an OCM reactor **3203,** which produces an OCM product stream **3204** comprising ethane (C₂H₆), ethylene (C₂H₄), carbon monoxide (CO) and/or carbon dioxide (CO₂), and heat. The OCM product stream can then be fed into an ethane conversion subsystem **3205** (e.g., a cracking vessel or an ethane cracker) in fluid communication with the OCM reactor. The ethane conversion subsystem can also receive an additional C₂H₆ stream **3206.** The ethane conversion subsystem can convert C₂H₆ (e.g., crack C₂H₆ to C₂H₄) with the aid of the heat liberated by the OCM reaction; this heat can also be used to crack the C₂H₆ in the additional C₂H₆ stream. A C₂H₄ product stream **3207** can then be directed from the ethane conversion subsystem into a separations module 3208 in fluid communication with the ethane conversion subsystem. The separations module can enrich products such as C₂H₄ in the product stream. The separations module can also oligomerize C₂H₄ to form compounds comprising three or more carbon atoms (C₃₊ compounds). An enriched product stream **3209** enriched in C₂H₄ and/or C₃₊ compounds can be recovered from the separations module. A lights stream **3210** comprising components such as hydrogen (H₂) (e.g., hydrogen generated from the cracking of C₂H₆) and CO and/or CO₂ can be recovered from the separations module and directed into a methanation reactor **3211** in fluid communication with the separations module. The methanation reactor can react H₂ with CO and/or CO₂ to form a methanated stream **3212** comprising CH₄. The methanated stream can then be directed into the OCM reactor to provide additional methane for the OCM process. In some cases, energy generated in the methane conversion section in the form of high pressure steam, high temperature steam, heat, electricity, heat transferred via gas-gas heat exchanger, heat transferred via gas-liquid heat exchanger, or other forms, can be used to provide all of the energy and power required to run the entire plant or system. In some cases, a cyclical system or process can operate with a carbon efficiency such as those discussed in this disclosure. For example, such a system or process can operate with a carbon efficiency of at least about 50%, at least about 55%, at least about 60%, at least about 65%, at least about 70%, at least about 75%, at least about 80%, at least about 85%, or at least about 90%. In some cases, such a system or process can operate with a carbon efficiency of between about 50% and about 85%, between about 55% and about 80%, between about 60% and about 80%, between about 65% and about 85%, between about 65% and about 80%, or between about 70% and about 80%. In some cases, such a system or process (or method) can operate such that a ratio of all carbon atoms output from the system as hydrocarbons to all carbon atoms input to the system is at least about 0.50, at least about 0.55, at least about 0.60, at least about 0.65, at least about 0.70, at least about 0.75, at least about 0.80, at least about 0.85, or at least about 0.90. In some cases, such a system or process can operate such that a ratio of all carbon atoms output from the system as hydrocarbons to all carbon atoms input to the system is between about 0.50 and about 0.85, between about 0.55 and about 0.80, between about 0.60 and about 0.80, between about 0.65 and about 0.85, between about 0.65 and about 0.80, or between about 0.70 and about 0.80.

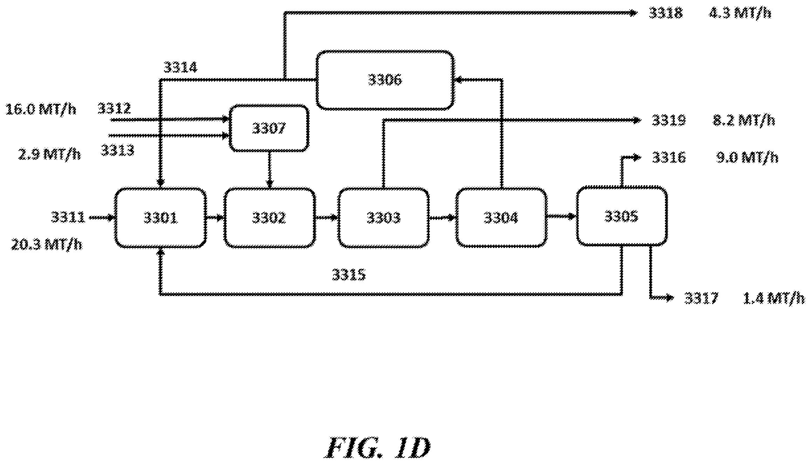

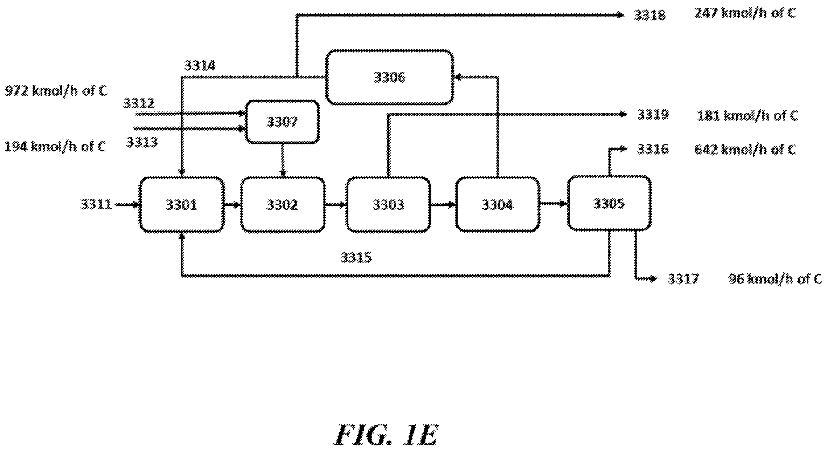

**FIG. 1D** and **FIG. 1E** show an exemplary process comprising an OCM unit **3301**, a process gas compressor **3302**, a process gas cleanup unit **3303**, a cryogenic separations unit **3304**, a fractionation unit **3305**, a methanation unit **3306**, and a sulfur-removal unit **3307**. An oxygen stream **3311** is fed into the OCM unit, along with a C₁ recycle stream **3314** from the methanation unit and a C₂ recycle stream **3315** from the fractionation unit. A natural gas stream **3312** and an ethane stream **3313** are fed into the sulfur removal unit. Output from the OCM unit and the sulfur removal unit are directed into the process gas compressor, and then into the process gas cleanup unit, which removes a CO₂ stream **3319**. The remaining product stream is directed into the cryogenic separations unit, where light components including H₂ and CO or CO₂ are directed into the methanation unit, and the remaining product stream, including ethylene and other C₂₊ compounds, is directed into the fractionation unit. The fractionation unit separates an ethylene stream **3316** and a C₃₊ compound stream **3317** comprising C₃ compounds, C₄ compounds, and C₅₊ compounds, as well as the C₂ recycle **3315** which is directed back to the OCM unit. The methanation unit converts the light components into methane, a first portion of which is recycled **3314** to the OCM unit and a second portion of which is output as sales gas **3318**. The operating flow rates for the input streams are as follows: 20.3 MT/h of oxygen **3311**, 16.0 MT/h of natural gas **3312**, and 2.9 MT/h of ethane **3313**. The operating flow rates for the output streams are as follows: 9.0 MT/h of ethylene **3316**, 1.4 MT/h of C₃₊ compounds **3317**, 4.3 MT/h of sales gas **3318**, and 8.2 MT/h of CO₂ **3319**. The corresponding carbon content of the input streams are 972 kmol/h of carbon in the natural gas stream **3312**, and 194 kmol/h of carbon in the ethane stream **3313**. The corresponding carbon content of the output streams are 642 kmol/h of carbon in the ethylene stream **3316**, 96 kmol/h of carbon in the C₃₊ compounds stream **3317**, 247 kmol/h of carbon in the sales gas stream **3318**, and 181 kmol/h of carbon in the CO₂ stream **3319**. Therefore, the amount of carbon input to the system is 1166 kmol/h, and the amount of carbon output from the system in hydrocarbon products (e.g., excluding CO₂) is 985 kmol/h, for a resulting carbon efficiency of 84.5%.

Reaction heat (e.g., OCM reaction heat) can be used to supply some, most, or all of the energy used to operate systems and perform processes of the present disclosure. In some examples, reaction heat can be used to supply at least about 50%, 55%, 60%, 65%, 70%, 75%, 80%, 85%, 90%, 95%, 96%, 97%, 98%, 99%, or 100% of energy for operating systems and performing processes of the present disclosure. For example, the reaction heat can be used to supply at least about 80% or 90% of all of the energy for operating systems or processes of the present disclosure. This can provide for an efficient, substantially self-contained system with reduced or even minimum external energy input.

### Integration of OCM Processes with Other Chemical Processes

There exists an infrastructure for chemical production throughout the world. This infrastructure is deployed on virtually every continent, addresses wide ranging industries, and employs a wide variety of different implementations of similar or widely differing technologies.

The present disclosure provides systems and methods for integrating OCM systems and methods with various chemical processes, such as methanol (MeOH) production, chlorine (Cl₂) and sodium hydroxide (NaOH) production (e.g., chloralkali process), vinylchloride monomer (VCM) production, ammonia (NH₃) production, processes having syngas (e.g., mixtures of hydrogen (H₂) and carbon monoxide (CO) in any proportion), or olefin derivative production.

As will be appreciated, the capital costs associated with each of the facility types described above can run from tens of millions to hundreds of millions of dollars each. Additionally, there are inputs and outputs, of these facilities, in terms of both energy and materials, which have additional costs associated with them, both financial and otherwise that may be further optimized in terms of cost and efficiency. Further, because different facilities tend to be optimized for the particularities (e.g., products, processing conditions) of the market in which they exist, they tend to be operated in an inflexible manner, in some cases without the flexibility or option to optimize for their given market. The present inventors have recognized surprising synergies when integrating OCM with the aforementioned chemical processes which can result in improved economics and/or operational flexibility.

In some cases, the OCM processes described herein are integrated with an olefin oligomerization process, such as an ethylene-to-liquids ("ETL") process as described in U.S. Patent Serial Nos. 14/099,614, and 14/591,850.

In some instances, the OCM process can be sized to fit the needs of an ethylene derivatives plant. Such a synergy can liberate the derivatives producer from being a merchant buyer of ethylene, allowing the producer more ethylene cost and supply certainty. Examples of ethylene derivatives include polyethylene, including low-density polyethylene (LDPE), linear low-density polyethylene (LLDPE), and high-density polyethylene (HDPE). Additional ethylene derivatives include ethylbenzene, styrene, acetic acid, vinylacetate monomer, ethylene dichloride, vinylchloride monomer, ethylene oxide and alpha olefins.

### Integration of OCM Processes with Methanol Processes

The OCM processes can be integrated with methanol production processes to realize unexpected synergies potentially including, but not limited to (a) additional methanol capacity with minimal or no modification to the methanol plant and (b) additional ethylene capacity with low investment and environmental footprint.

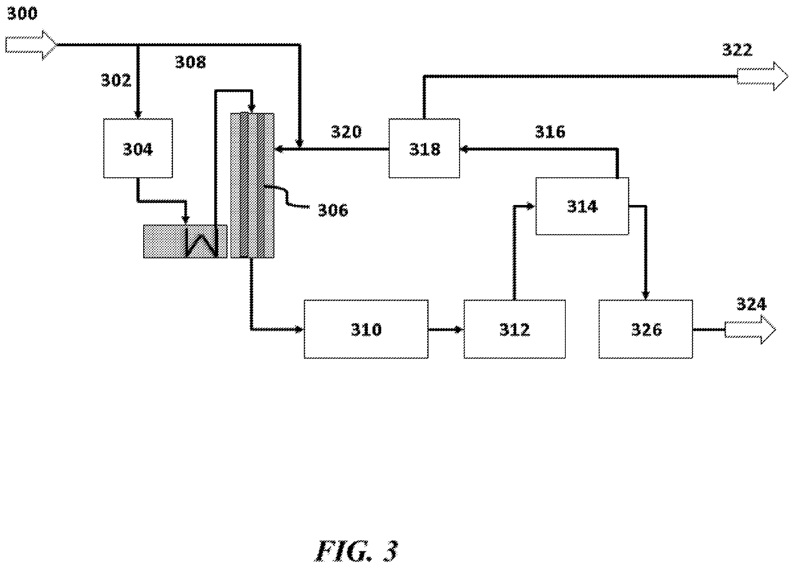

**FIG. 3** shows an example of a block flow diagram of a methanol plant (e.g., a traditional methanol process, recognizing that alternate embodiments are allowed and details have been emitted for clarity). As shown, natural gas **300** can be used for feed and fuel for the process. The feed **302** (e.g., natural gas providing the carbon atoms for the methanol product) can have sulfur-containing compounds removed in a de-sulfurization module **304** before being fed into a steam methane reformer (SMR, entire gray shaded unit) **306**. The SMR can also accept natural gas as fuel **308** (e.g., natural gas providing energy for the methanol plant), which does not necessarily have to be de-sulfurized. The effluent of the steam methane reformer is syngas, which can have heat recovered in a heat recovery module **310** and compressed in a compression module **312**. Compressed syngas can be feed into the synthesis module **314** where conversion to methanol occurs. One suitable methanol synthesis module can have a catalyst that is a mixture of copper, zinc, and alumina, and operates at a pressure between about 50 and about 100 atmospheres and a temperature of about 250 °C. The production of syngas produces 3 moles of H₂ per mol of CH₄, while the stoichiometry of methanol formation from syngas consumes only 2 moles of H₂. Thus, excess H₂ (and un-reacted CH₄) can be purged **316** from the synthesis module and separated in a gas separation module **318** (e.g., a pressure swing adsorber). The separation module can produce additional fuel **320** for the SMR and a H₂ co-product **322**. The methanol product **324** can be enriched (e.g., by a distillation module **326**). In some cases, the excess H₂ is used as fuel (not shown).

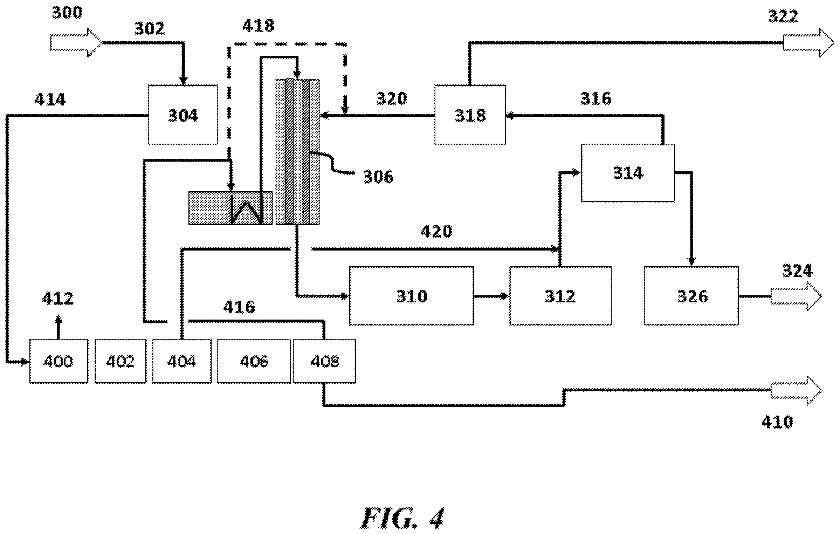

A combined process that integrates OCM with methanol production is shown in **FIG. 4**, where like numerals represent like elements. The OCM portion of the combined process can accept the de-sulfurized natural gas feedstock **414** and include an OCM reaction module **400**, a process gas compression module **402**, a CO₂ removal module (e.g., process gas cleanup) **404**, a drying module **406** and a separations module (e.g., a cryogenic de-methanizer) **408**. In some cases, the separation module produces the C₂₊ compounds **410.** The C₂₊ compounds can be further refined, and/or sent to a cracker (e.g., to the separation section of a cracker). Note that the OCM process does not require a methanation module. The OCM reaction can produce highpressure super-heated (HPSH) steam **412** that can be used in the process and/or to produce power using a steam turbine.

Continuing with **FIG. 4****,** the OCM portion of the process can produce a stream of methane that was not converted to C₂₊ compounds **416** in the OCM reaction. This stream **416** can have H₂ and CO in addition to methane and can be used as the feed to the methanol production process (e.g., at the SMR) and/or as fuel to the process (dashed line) **418**. The stream of CO₂ **420** from the OCM process can also be used in the methanol synthesis module **314** to produce one mole of methanol and one mole of water from one mole of CO₂ and 3 moles of H₂. The water co-product can be removed in the distillation module **326**.

The combined OCM-methanol process has considerable economic and environmental benefits. In some cases, CO₂ from OCM **420** can be used to re-balance the makeup gas to the synthesis module and convert some or all of the excess H₂ to methanol (e.g., the flow-rate of stream **322** can be zero or very small in comparison to the flow rate without OCM integration). Furthermore, the reformer **306** capacity can be automatically increased due to the "pre-formed" nature of the OCM demethanizer overhead **416** stream (e.g., already contains some H₂ and CO). This can be useful for replacing a mixed feed coil. In some instances, the only cost associated with the production of extra methanol due to OCM integration is the loss in value of the H₂ co-product **322** in situations where that stream is actually monetized or monetizable. Such integration schemes can result in improved efficiency of an existing methanol system, for example by using excess H₂ by reacting it with CO₂ produced from an OCM unit to produce a more valuable methanol product. Depending on the capacity of the OCM process, an integrated OCM-methanol system can be pushed to a low emission, high carbon efficiency process.

When retrofitting an existing methanol plant, the OCM process can be sized to the desired amount of extra methanol production. From the OCM perspective, building an OCM process to be integrated with a methanol plant can require significantly less capital than building a stand-alone OCM process, e.g., due to reducing or eliminating the need for fractionation and methanation equipment. The OCM process can also use the utilities of the existing methanol plants, such as steam. In some cases, the combined process produces zero or a minimal amount of NOₓ and SOₓ compounds.

The combined OCM-methanol process can be about 100% carbon efficient (e.g., with reference to **FIG. 4****,** all of the carbon atoms input to the process **300** end up in the methanol **324** or the C₂₊ compounds **410**). In some cases, the combined process is less than 100% carbon efficient, e.g., greater than or equal to about 99%, greater than or equal to about 98%, greater than or equal to about 97%, greater than or equal to about 96%, greater than or equal to about 95%, greater than or equal to about 93%, greater than or equal to about 90%, greater than or equal to about 85%, greater than or equal to about 80%, or greater than or equal to about 75% carbon efficient.

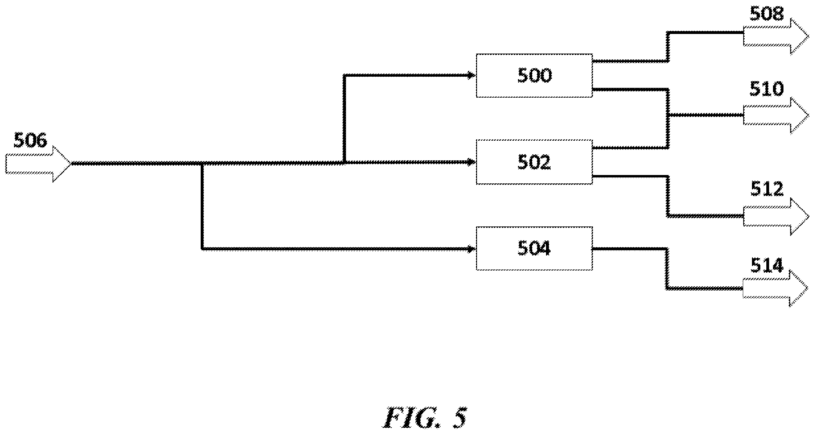

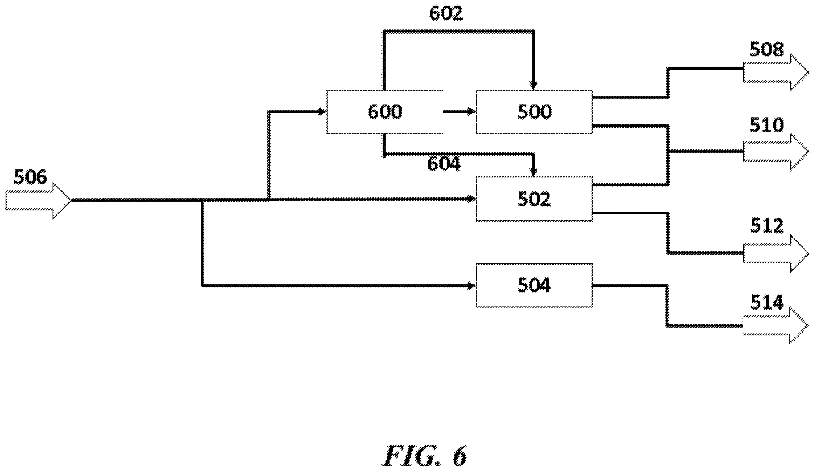

In some cases, with reference to **FIG. 5**, methanol plants **500** are located in proximity to crackers **502** and/or other processes **504** that use natural gas (e.g., within 1, 5, 10, 20, 50, 100, 200 miles or more). In some cases, these processes share a piping infrastructure and/or can access a piping infrastructure for transporting natural gas, ethylene, hydrogen and other chemicals. These processes can convert the natural gas **506** into a combination of methanol **508**, hydrogen **510**, ethylene **512**, and other products **514**. OCM can be integrated with any combination of these processes (e.g., **500, 502** and **504)** in a number of ways as shown in **FIG. 6****,** **FIG. 7** and **FIG. 8****.**

**FIG. 6** shows a "minimum revamp case" where an OCM process **600** accepts natural gas **506** and provides CO₂ **602** to a methanol process **500** and crude ethylene **604** to a cracker **502**. The ethylene can be refined to a finished product (e.g., polymer grade ethylene) **512** using the fractionation capacity of the cracker. In this case, the OCM process can be sized to accept an amount of natural gas that is substantially equivalent to the methanol plant natural gas input (e.g., about 60 to 70 MMSCFD). This OCM capacity can result in about 25-30 kTa additional ethylene and about 15% to 20% additional methanol produced. In some cases, for the minimum revamp case, the only capital investment is for the OCM unit **600** and in some cases mixed feed coil replacement in the SMR.

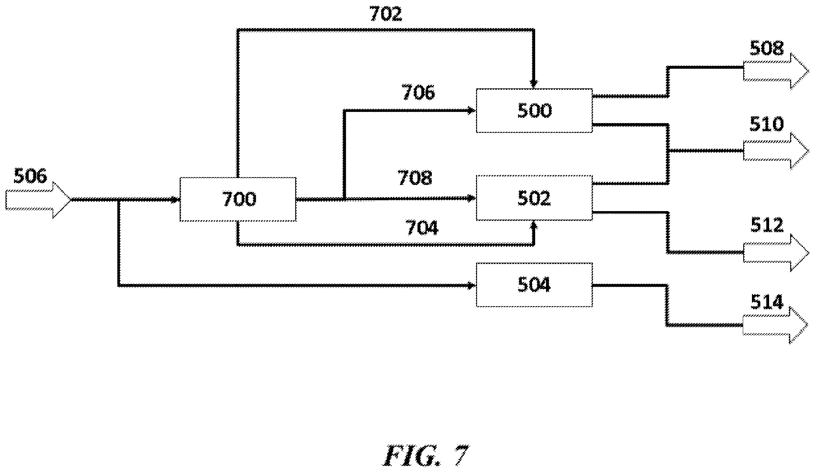

**FIG.** 7 shows a "medium revamp case" where an OCM process **700** accepts natural gas **506** and provides CO₂ **702** to a methanol process **500** and crude ethylene **704** to a cracker **502**. In this case, the OCM process can be sized to accept an amount of natural gas that is substantially equivalent to the methanol plant natural gas input **706** and cracker fuel input **708** (e.g., about 140 to 150 MMSCFD). This OCM capacity can result in about 60-80 kTa additional ethylene and about 30% to 40% additional methanol produced. In some cases, for the medium revamp case, capital investment is needed for the OCM unit **700** and methanol debottlenecking (e.g., reformer, syngas compressor, synthesis module and topping column).

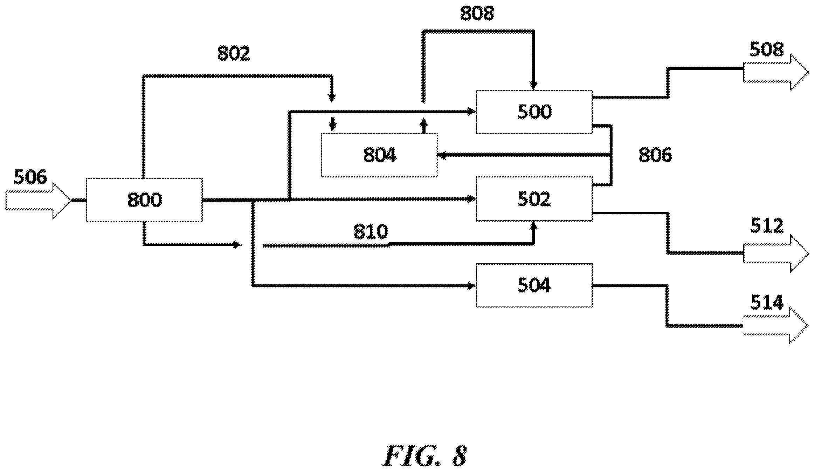

**FIG. 8** shows a "maximum efficiency revamp case" where the size of the OCM process is not constrained. For example, all of the natural gas entering an entire petrochemical complex can be skimmed. An OCM process **800** accepts natural gas **506** and provides CO₂ **802** to a new methanol synthesis module 804. In some cases, the new methanol synthesis module 804 accepts H₂ 806 from various sources including an existing methanol process 500 and/or a cracker 502. The new methanol synthesis module 804 can provide crude methanol 808 to the existing methanol process for refining to a methanol product 508. As in the other revamp scenarios, crude ethylene 810 can be refined in a cracker 502. In some cases, the OCM results in about 150-200 kTa additional ethylene, the integration results in about 60% to 70% additional methanol produced. In some cases, for the maximum efficiency revamp case, capital investment is needed for the OCM unit, a new methanol synthesis module (fed with the excess H₂ across the entire complex and CO₂ from OCM) and in some cases debottlenecking of methanol distillation. The various revamp cases are not mutually exclusive and can be designed as successive project phases. In addition, larger capacity plants can be combined with larger methanol production plants.

### Integration of OCM Processes with Chloralkali Processes

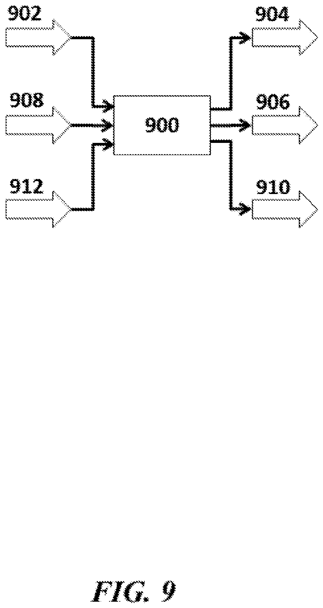

With reference to **FIG. 9**, the chloralkali process **900** is an industrial process for the electrolysis of sodium chloride (NaCl) **902** to produce chlorine gas (Ch) **904** and sodium hydroxide (NaOH) **906**. The process is typically conducted with an aqueous solution of sodium chloride (NaCl) (e.g., the process uses water **908**) and produces a hydrogen (H₂) **910** co-product. Other chloride compounds can be used, such as lithium chloride (LiCl), potassium chloride (KCl), calcium chloride (CaCl₂), and magnesium chloride (MgCl₂), and hydrates thereof. The chloralkali process consumes a considerable amount of electrical power **912**. There are three chloralkali methods currently used in industry, referred to as membrane plants, diaphragm plants, and mercury plants. New electrochemical cells and processes are being developed that, for example, use a metal chloride intermediate, such as described in U.S. Patent Application Serial Nos. 12/989,785, 12/721,545, 12/375,632, and 12/541,055. Each type of chloralkali process can be integrated with OCM to realize surprising synergies described herein.

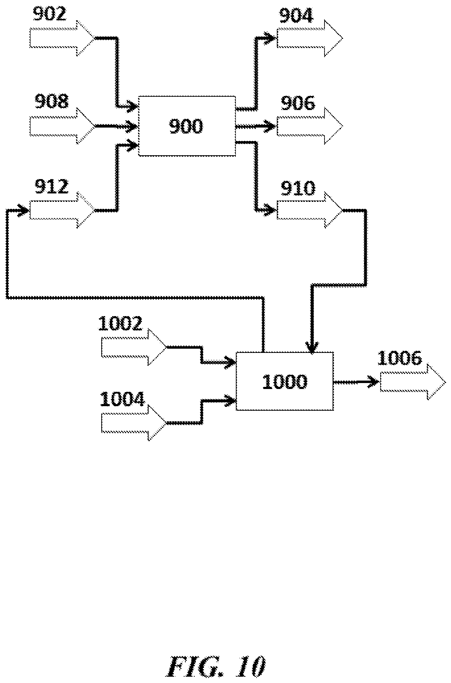

**FIG. 10** shows a schematic illustration of an OCM process **1000** integrated with a chloralkali process **900**. The OCM process, consumes oxygen (O₂) **1002** and methane **1004** (e.g., natural gas) and produces C₂₊ compounds such as ethylene **1006**. The OCM process can accept H₂ **910** from the chloralkali process (e.g., at the methanation module **110** as shown in **FIG. 1A**) for conversion of CO and/or CO₂ to additional methane for recycle to the OCM reactor. The OCM process is exothermic and the heat of reaction can be converted to electricity **912** (e.g., cogeneration) for use in the chloralkali process.

### Integration of OCM Processes with EDC and/or VCM Process

The present disclosure recognizes certain unexpected synergies that can be achieved by integrating OCM with the production of vinylchloride monomer (VCM) and/or ethylene dichloride (EDC) (e.g., EDC/VCM process). This is because the EDC/VCM process uses ethylene as a feedstock, but does not require polymer-grade ethylene. Therefore, the OCM process does not require significant capital and operating expense associated with purifying ethylene.

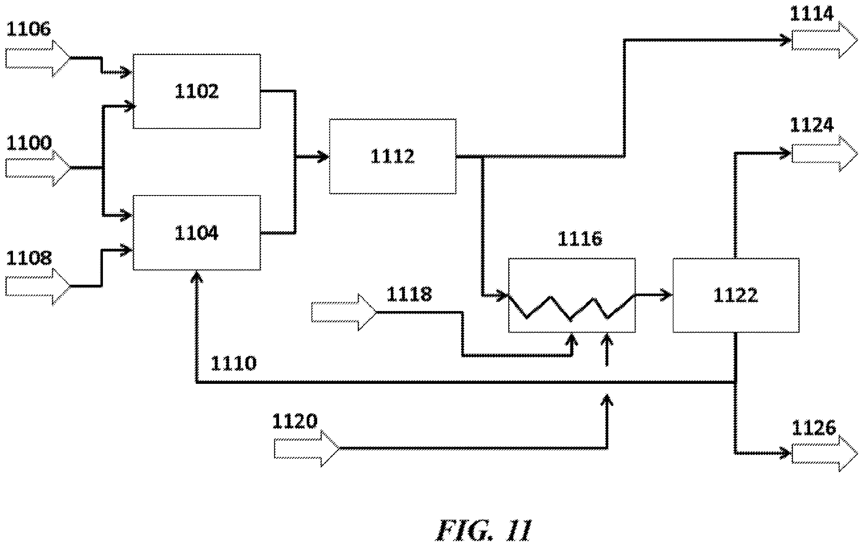

With reference to **FIG. 11**, the ethylene **1100** can be provided by OCM (not shown). The ethylene can be about 99.99%, about 99.95%, about 99.9%, about 99.5%, about 99%, about 97%, about 95%, about 93%, about 90%, about 85%, about 80%, about 75%, or about 70% pure on a mass basis. In some cases, the ethylene is less than about 99.99%, less than about 99.95%, less than about 99.9%, less than about 99.5%, less than about 99%, less than about 97%, less than about 95%, less than about 93%, less than about 90%, less than about 85%, less than about 80%, less than about 75%, or less than about 70% pure on a mass basis. In some cases, the ethylene is greater than about 70%, greater than about 75%, greater than about 80%, greater than about 85%, greater than about 90%, greater than about 93%, greater than about 95%, greater than about 97%, greater than about 99%, greater than about 99.5%, greater than about 99.9%, greater than about 99.95%, or greater than about 99.99% pure on a mass basis.

Continuing with **FIG. 11**, the ethylene **1100** can be added to a direct chlorination reactor **1102** and/or an oxy-chlorination reactor **1104**. The direct chlorination reactor **1102** uses chlorine gas (Cl₂) **1106** as a reactant and the oxy-chlorination reactor **1104** uses oxygen (O₂) **1108** and hydrochloric acid (HCl) **1110** as reactants. The HCl can be produced in the process and recycled to the oxy-chlorination reactor **1104**. A first separations module **1112** can be used to enrich an EDC product **1114**. A portion of the EDC can be cracked in a furnace **1116** using, for example, energy derived from natural gas **1118** and/or H₂ **1120**. The cracked EDC can be separated in a second separations module **1122** to provide a VCM product stream **1124** and HCl **1126**, which can be recycled **1110** to the oxy-chlorination reactor **1104**.

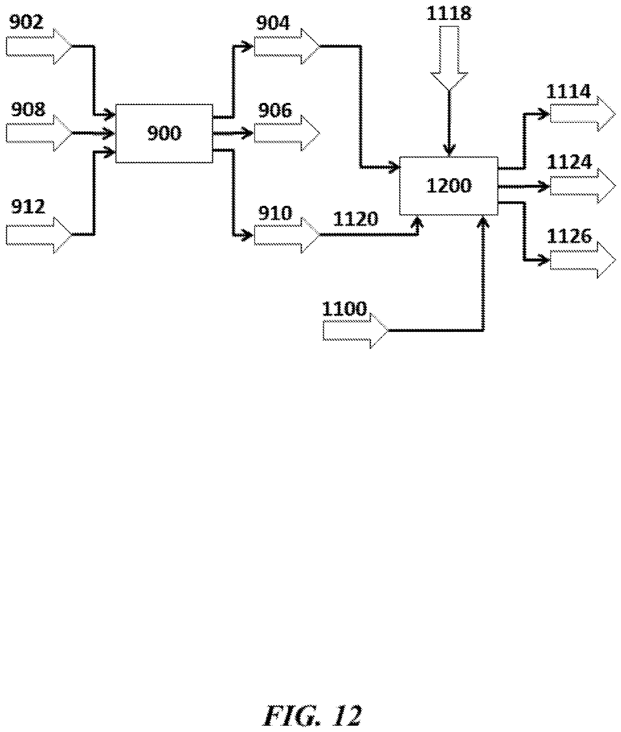

Some chloralkali process are integrated with the production of vinylchloride monomer (VCM) and/or ethylene dichloride (EDC). As shown in **FIG. 12**, some of the Cl₂ **904** produced by the chloralkali process **900** can be used in the EDC/VCM process **1200** (e.g., in the direct chlorination reactor **1102**). Also, some or all of the H₂ **910** produced by the chloralkali process **900** can be used in the EDC/VCM process **1200** (e.g., as fuel **1120** for the EDC cracking furnace **1116**). Additional fuel for the EDC cracking furnace can be derived from natural gas **1118.** The process consumes ethylene **1100** and some of the products of the combined chloralkali and EDC/VCM process include EDC product **1114,** VCM **1124** and HCl **1126**.

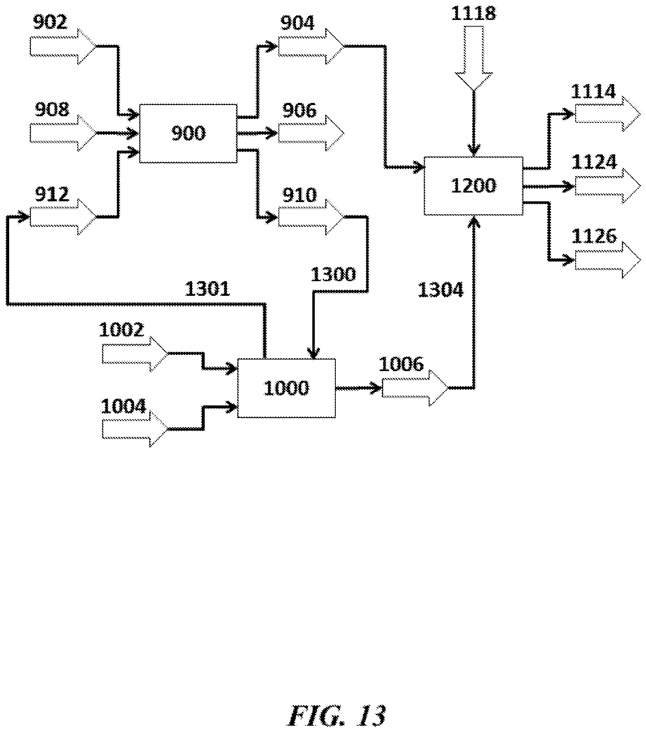

In some instances, as shown in **FIG. 13**, OCM can be integrated with both an EDC/VCM process and a chloralkali process. In this case, the chlorlkali process **900** provides H₂ **1300** to the OCM process, the OCM process provides electrical power **1302** to the chloralkali process, and the OCM process provides ethylene **1304** to the EDC/VCM process **1200**.

In some cases, a modified chloralkali process is integrated with a modified EDC production process in which Cl₂ is not produced as an intermediate. Instead, a metal chloride solution can be produced (e.g., CuCl₂) as the intermediate, for example as described in U.S. Patent Application Serial No. 14/446,791. OCM can also be integrated with these facilities as described herein.

The processes of the present disclosure can take advantage of the synergies made possible by OCM integration to chloralkali, EDC, or VCM producing units. An OCM unit can be a good fit between inputs and outputs of the two processes; OCM can produce ethylene and power, which can be the main inputs to chloralkali, EDC, or VCM processes. Chloralkali processes can produce hydrogen as a main co-product, which can be utilized in an OCM unit (rather than being combusted or vented) to reduce or eliminate CO₂ emissions and push carbon efficiency towards or up to 100%. EDC processes can operate with non-polymer-grade ethylene (alkanes are inert in EDC processes), so the separations unit of an OCM unit can produce chemical grade ethylene, which can result in a reduced capital expenditure (capex). Additionally, typical EDC scale can match small scale OCM implementations.

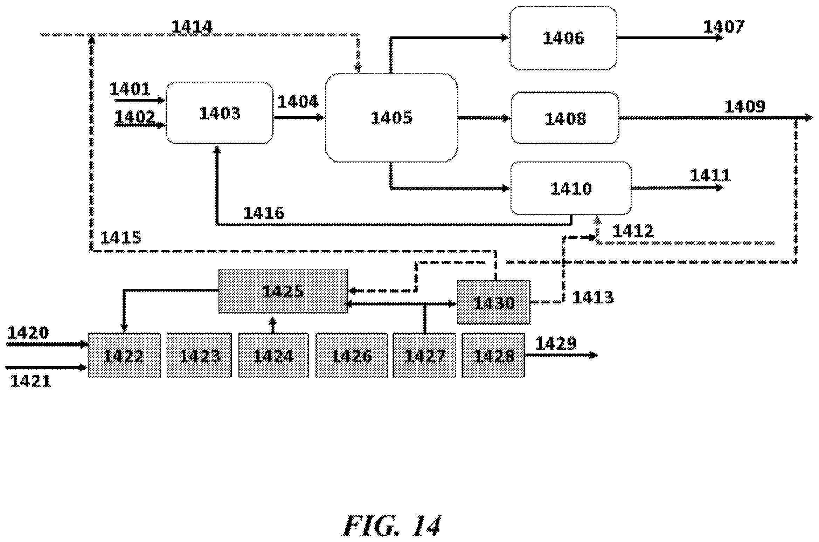

**FIG. 14** shows an example of a process integrating OCM with a diaphragm-type chloralkali process. The production capacity of the chloralkali process is at least about 300,000 tons per year (300 kTa) of chlorine. The production capacity of the OCM process is at least about 100,000 tons per year (100 kTa) of ethylene. Co-generation with the OCM process can produce about 100-120 ton/hr of steam and about 80-120 MW of power.

Salt 1401 and water 1402 are fed to a brine saturation unit 1403, and purified brine 1404 is then fed into an electrolysis unit 1405. The electrolysis of purified brine in the chloralkali process uses power 1414 (e.g., up to about 2970 kWh per ton of Cl₂ produced); at least a portion of this power can be provided 1415 from co-generation with the OCM process (e.g., about 80-120 MW). A chlorine product stream can be subjected to treatment and liquefaction 1406 before being output as chlorine product 1407 (e.g., at least about 300 kTa). A hydrogen stream can be subjected to cooling and oxygen removal 1408 before further use; hydrogen 1409 (e.g., at least about 8400 kTa or at least about 950 kg/hr) can be directed into a methanation unit in the OCM process, for example. A caustic soda product stream 1411 (e.g., 50% caustic soda) can be produced (e.g., about 338.4 kTa) after concentration and cooling 1410. A reclaimed salt stream 1416 can be recycled to the brine saturation unit. The cooling process can use steam 1412 (e.g., up to about 610 kWh per ton of Cl₂), at least a portion of which can be provided 1413 from co-generation 1430 with the OCM process (e.g., about 100-120 ton/hr). It is assumed that 1 ton of steam is 250 kWh at 19 bar. The processes are integrated with respect to electrical power, hydrogen and steam. Natural gas 1420 and ethane 1421 can be fed into an OCM reactor 1422 with other reagents and reacted in an OCM process. Post-bed cracking 1423 can be employed to produce additional ethylene. CO₂ can be removed in a CO₂ removal unit 1424 and fed into a methanation unit 1425. The OCM product stream can be further processed in a drying unit 1426, a de-methanizer unit 1427, and a C₂ hydrogenation unit 1428, producing an ethylene stream 1429.

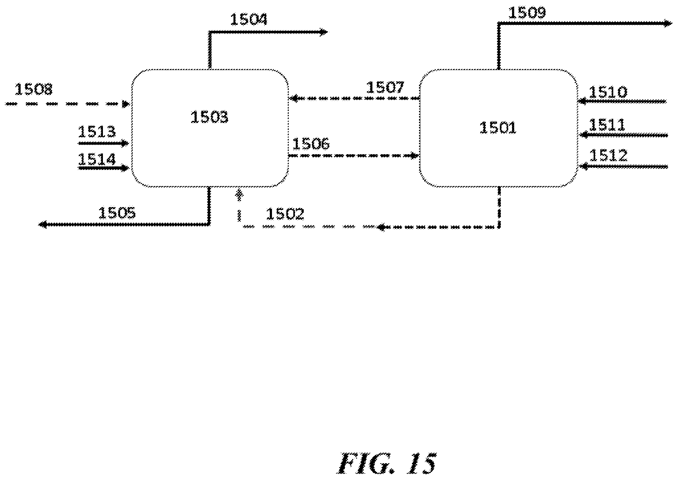

**FIG. 15** shows a material and energy balance for the process shown in **FIG. 14**. All of the electrolytic hydrogen is used in the OCM unit 1501. The OCM process provides a portion of the steam 1502 used by the electrolysis unit 1503 (e.g., chloralkali process). The electrolysis unit produces at least about 300 kTa of chlorine 1504 and at least about 338.4 kTa of caustic soda 1505, as well as at least about 950 kg/hr of hydrogen 1506 which is fed into the OCM unit. The chloralkali process receives about 80-120 MW of power 1507 from the OCM process, and additional power 1508 of at least about 104 MW from other sources. The chloralkali process also receives salt 1513 and water 1514.The OCM unit produces at least about 100 kTa ethylene 1509 (e.g., at least about 0.3 tons of ethylene per ton of C1₂ produced by the chloralkali process), as well as at least about 85 ton/hr steam (e.g., up to about 610 kWh per ton of Cl₂) which is fed into the electrolysis unit. The OCM unit consumes about 40-50 MMSCFD of natural gas 1510, about 15-18 MMSCFD of oxygen 1511, and about 6-9 MMSCFD of ethane 1512.

### Integration of OCM Processes with an Ammonia Process

The present disclosure provides techniques that can advantageously employ certain unexpected synergies that can be achieved by integrating OCM with the production of ammonia (NH₃). In some cases, an existing ammonia process is retrofitted with an OCM process. These synergies can include increasing the capacity of a reforming portion of an ammonia process, in some cases without modification of the steam methane reformer and/or secondary reformer. In some cases, such a reforming capacity expansion can be achieved without overburdening other unit operations leading up to the ammonia synthesis module (e.g., the "synloop"). Therefore, the addition of an OCM process to an ammonia production process can be performed without the significant capital and operating expense that can be associated with purifying ethylene.

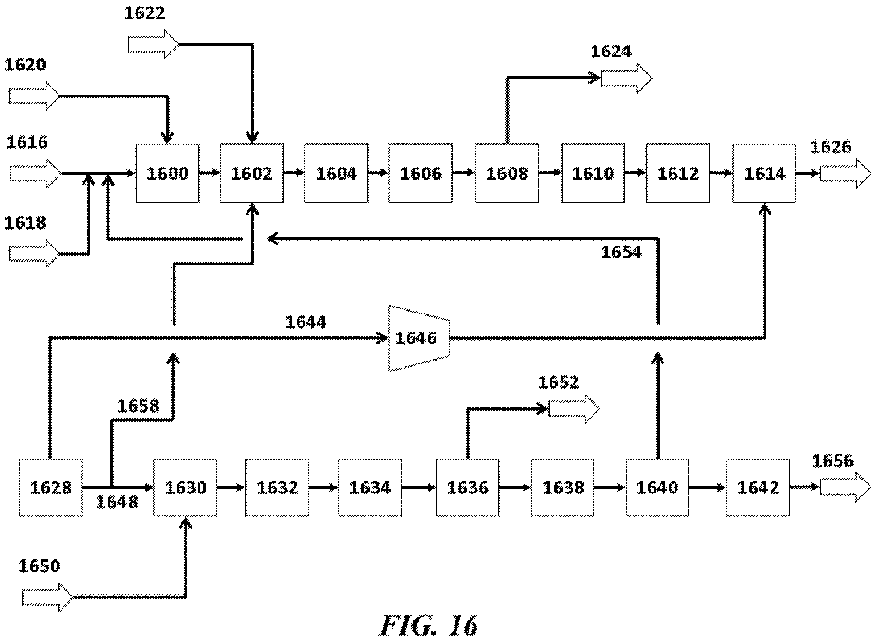

With reference to **FIG. 16**, an ammonia process can comprise a steam methane reformer **1600**, a secondary reformer **1602**, a heat recovery module **1604**, a water-gas shift conversion unit **1606**, a CO₂ separation module **1608**, a methanation reactor **1610**, a syngas compressor **1612** and an ammonia synthesis and separation module **1614**. The ammonia synthesis module can be an implementation of the Haber-Bosch catalytic process.

Following the ammonia process, the steam methane reformer **1600** can accept natural gas (e.g., as feedstock) **1616** and combine it with steam **1618**. The feedstock can enter the tubeside of the SMR, for example at a temperature of about 500 °C. A large amount of heat can be supplied to the tubes of the SMR, for example via combustion of natural gas fuel **1620** in the radiation section of the SMR, in order to heat up the reacting feed (e.g., to a temperature from about 740 to about 800 °C) and sustain an endothermic reforming reaction that produces syngas (e.g., via the reaction CH₄ + H₂O ←→ CO + 3H₂, which heat of reaction can also be supplied by the natural gas fuel **1620**). Because reforming is an equilibrium reaction, a certain portion of the methane may not be converted to syngas in the SMR (e.g., about 8-15%). The SMR effluent can be directed to a secondary reformer **1602** where air **1622** is added to reduce the methane (CH₄) concentration to about 0.3-1.2%, such as via a combination of combustion and reforming reactions. At this point, the temperature of the stream can be as high as about 900-1000 °C; a heat recovery module **1604** can be used to lower the temperature and recover energy, such as via generation of high pressure superheated steam. The cooled product then can then be directed to a water-gas shift reactor **1606** to produce more hydrogen (e.g., via the reaction CO + H₂O ←→ CO₂ + H₂). At this point, the ratio of H₂ to N₂ can be about 3, which can match the reaction stoichiometry for ammonia production. CO₂ can then be removed **1624** in a separation module **1608**, leaving about 5-50 ppm CO₂ and about 0.1-0.4% CO. CO₂ and CO can be strong poisons to ammonia synthesis catalysts, so residual amounts of CO₂ and CO can be converted to methane (which is inert in the ammonia synthesis reaction) in a methanation reactor **1610**. A syngas compressor **1612** and an ammonia synthesis and separation module **1614** can be used to complete the process and produce ammonia **1626**. Note that for clarity, various streams and units, such as ammonia purification, may not have been shown or described.

In an ammonia process, the extent of reaction in the secondary reformer **1602** can be limited by the amount of air **1622**, as the nitrogen (N₂) from this air stream can be the source of N₂ for the production of ammonia. However, integrating and/or retrofitting an ammonia process with an OCM process can obviate this limitation, along with providing additional benefits, including those discussed herein.

With reference to **FIG. 16**, the OCM process can comprise an air separation unit (ASU) **1628**, an OCM reactor **1630**, a heat recovery module **1632**, a compression module **1634**, a CO₂ removal unit **1636**, a dryer module **1638**, a de-methanizer module **1640**, and a fractionation module **1642**. Following the OCM process, the ASU can separate air into a nitrogen stream **1644,** which can be fed to the ammonia process to provide a source of clean N₂ reactant (e.g., not having oxygenated compounds such as CO or CO₂). In some cases, other processes (e.g., separations) can be used to provide air and nitrogen. For example, a pressure swing adsorption (PSA) unit can be used to provide O₂ and N₂. The ammonia synthesis module **1614** can operates at about 80 to 200 bar pressure. The nitrogen stream **1644** can be compressed to operating pressure in an auxiliary compressor **1646** or in the syngas compressor of the ammonia process **1612.** The oxygen (O₂) **1648** produced by the ASU **1628** can be supplied to both the OCM reactor **1630** and the secondary reformer of the ammonia process **1602** (e.g., offsetting or supplementing O₂ from air **1622**). Continuing with the OCM process, the oxygen can be reacted with methane **1650** (e.g., from natural gas) to produce ethylene. Pressure can increase, and heat, CO₂ and water can be recovered in a series of units (e.g., **1632, 1634, 1636** and **1638** in any order). In some cases, CO₂ from the ammonia process **1624** and/or OCM process **1652** can be used in processes including but not limited to methanol, chloralkali, urea, and combinations thereof. The overhead stream **1654** from the de-methanizer **1640** can comprise un-converted methane from the OCM process which can be used to supplement and/or offset natural gas to the SMR of the ammonia process **1600**. Since this overhead stream **1654** can have H₂ (e.g., about 10%) and CO (e.g., about 1.5%), the stream is already partially reformed. The bottoms from the de-methanizer **1640** can be sent to the fractionation module **1642** to produce ethylene product **1656.**

Integrating and/or retrofitting an ammonia process with an OCM process can result in additional H₂ and/or NH₃ produced (e.g., at least about 5%, at least about 10%, at least about 15%, at least about 20%, at least about 25%, at least about 30%, at least about 35%, or at least about 40% additional H₂ and/or NH₃ compared to an ammonia process without OCM). This capacity expansion can emerge from any combination of a number of effects, such as: (a) the OCM process can supply the ammonia process with some partially reformed material (i.e., about 10% H₂ and about 1.5% CO in the de-methanizer overhead **1654**); (b) in contrast to natural gas, the de-methanizer overhead **1654** can lack "superior hydrocarbons" (e.g., C₂₊ alkanes), therefore the temperature threshold at which coking may occur can be higher and accordingly the SMR inlet temperature can be raised (e.g., raised from about 500 °C to about 550 °C or about 600 °C), allowing the heat supplied in the SMR radiation section to go toward the heat of reaction rather than providing a temperature increase, and thus increasing the syngas production performed by the SMR unit itself; and/or (c) supplying clean nitrogen (N₂) **1644** can break the stoichiometric limit of air **1622** as the sole nitrogen source, this coupled with O₂ supplementation **1658** can allow relatively more reforming to be carried out in the secondary reformer **1602**, allowing a higher amount of CH₄ slippage from the SMR (e.g., about 15-25% rather than 8-15% of unconverted methane).

In some cases, the process units between reforming and ammonia synthesis do not need to be de-bottlenecked or capacity expanded because, while extra H₂ is produced, the N₂ enters the process after these steps (i.e., at **1644** rather than with the air **1622**), so the total process flow is relatively unchanged.

In some cases, the ammonia synloop **1614** requires expansion in a revamp, however this is a relatively low capital item in comparison to the rest of the ammonia process units and such revamp results in increased ammonia product **1626**.

The systems and methods of the present disclosure can be nitrogen-efficient and/or energy-efficient. In some cases, the systems or methods of the present disclosure have a nitrogen efficiency of at least about 50%, at least about 55%, at least about 60%, at least about 65%, at least about 70%, at least about 75%, at least about 80%, at least about 85%, or at least about 90%. In some cases, a system of the present disclosure or method for use thereof has a ratio of all nitrogen atoms output from the system as nitrogen products to all nitrogen atoms input to the system of at least about 0.4, at least about 0.50, at least about 0.55, at least about 0.60, at least about 0.65, at least about 0.70, at least about 0.75, at least about 0.80, at least about 0.85, at least about 0.90, or at least about 0.95.

In some cases, the systems or methods of the present disclosure have a nitrogen efficiency of between about 50% and about 85%, between about 55% and about 80%, between about 60% and about 80%, between about 65% and about 85%, between about 65% and about 80%, or between about 70% and about 80%. In some cases, a system of the present disclosure or method for use thereof has a ratio of all nitrogen atoms output from the system as nitrogen products to all nitrogen atoms input to the system of between about 0.50 and about 0.85, between about 0.55 and about 0.80, between about 0.60 and about 0.80, between about 0.65 and about 0.85, between about 0.65 and about 0.80, or between about 0.70 and about 0.80.

In some instances, the nitrogen efficiency is at least about 50%, at least about 55%, at least about 60%, at least about 65%, at least about 70%, at least about 75%, at least about 80%, at least about 85% or at least about 90%. In some instances, the nitrogen efficiency is between about 50% and about 85%, between about 55% and about 80%, between about 60% and about 80%, between about 65% and about 85%, between about 65% and about 80%, or between about 70% and about 80%. In some instances, a system of the present disclosure or method for use thereof has a ratio of all nitrogen atoms output from the system as nitrogen products to all nitrogen atoms input to the system of at least about 0.60, at least about 0.65, at least about 0.70, at least about 0.75, at least about 0.80, at least about 0.85 or at least about 0.90. In some instances, a system of the present disclosure or method for use thereof has a ratio of all nitrogen atoms output from the system as nitrogen products to all nitrogen atoms input to the system of between about 0.50 and about 0.85, between about 0.55 and about 0.80, between about 0.60 and about 0.80, between about 0.65 and about 0.85, between about 0.65 and about 0.80, or between about 0.70 and about 0.80.

### Integration of OCM Processes with a Methanol to Propylene (MTP) Process

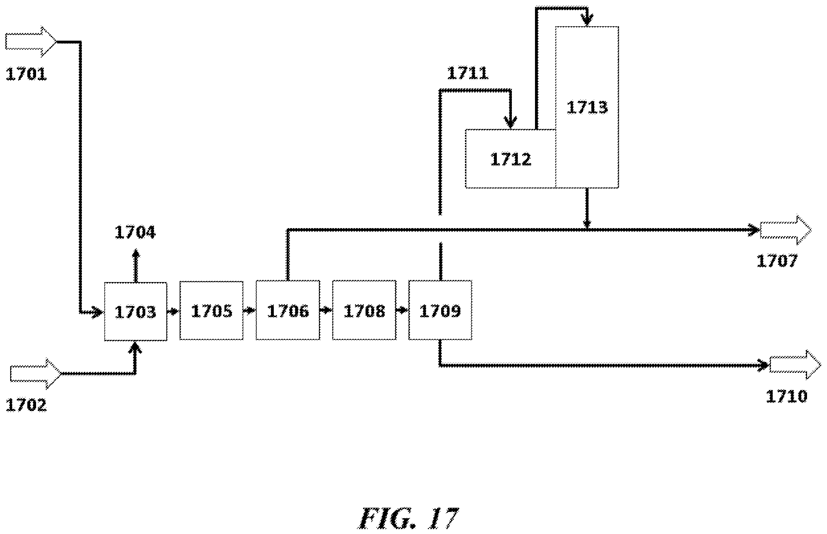

**FIG. 17** shows an exemplary OCM process for integration with a methanol to propylene (MTP) process. Natural gas 1701 and oxygen 1702 are fed into an OCM reactor 1703. High pressure superheated (HPSH) steam 1704 is produced from the OCM unit. The OCM product stream is fed into a post-bed cracking unit 1705, and then into a CO₂ removal unit 1706. Recovered CO₂ is directed for use in a balanced syngas stream 1707. The OCM product stream is further directed through a drying unit 1708 and a de-methanizer unit 1709. C₂₊ compounds 1710 are recovered from the de-methanizer, while unconverted methane and other light components including H₂ and CO 1711 can be directed to a reformer (e.g., a steam methane reformer) 1712 and 1713. The components are then added to the balanced syngas stream.

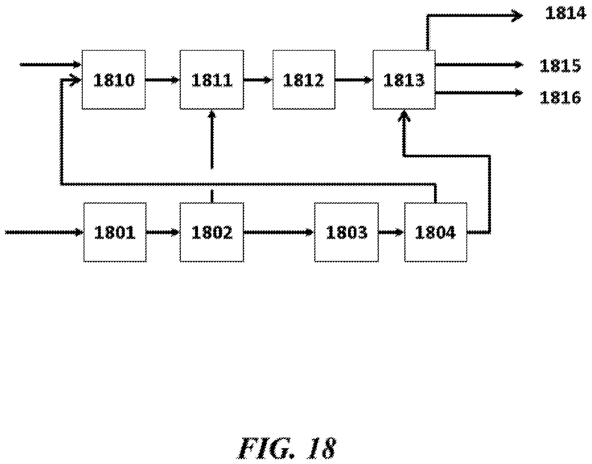

**FIG. 18** shows an exemplary integration scheme for an OCM process and an MTP process. Natural gas and oxygen are fed into an OCM unit 1801 with a post-bed cracking region. The OCM product stream is then processed in a CO₂ removal unit 1802, a drying unit 1803, and a de-methanizer unit 1804. In parallel, a methane stream (e.g., natural gas) is fed into a syngas unit 1810, along with a methane stream from the de-methanizer unit. The syngas stream is fed into a methanol synthesis unit 1811, along with CO₂ from the CO₂ removal unit. Methanol from the methanol synthesis unit is then fed into a methanol-to-propylene synthesis unit 1812, and the MTP product stream is fed into a recovery unit 1813. The C₂₊ product stream from the de-methanizer unit is also fed into the recovery unit. An ethylene stream 1814, a propylene stream 1815, and a C₄₊ compounds stream 1816 are recovered from the recovery unit.

### Integration of OCM Processes with a Liquid Natural Gas (LNG) Process

OCM and/or ETL processes can be integrated with liquid natural gas (LNG) processes.

For example, an LNG process can be integrated with OCM and ETL processes for fuel production. Such a process can convert methane, ethane, and optionally propane into fuel such as high-octane gasoline. Capital expenditure (CapEx) can be reduced due to synergies and overlap in needed equipment, such as product separations equipment. A fuel product, such as gasoline, can be mixed with condensate from the LNG process or separated via a dedicated column.

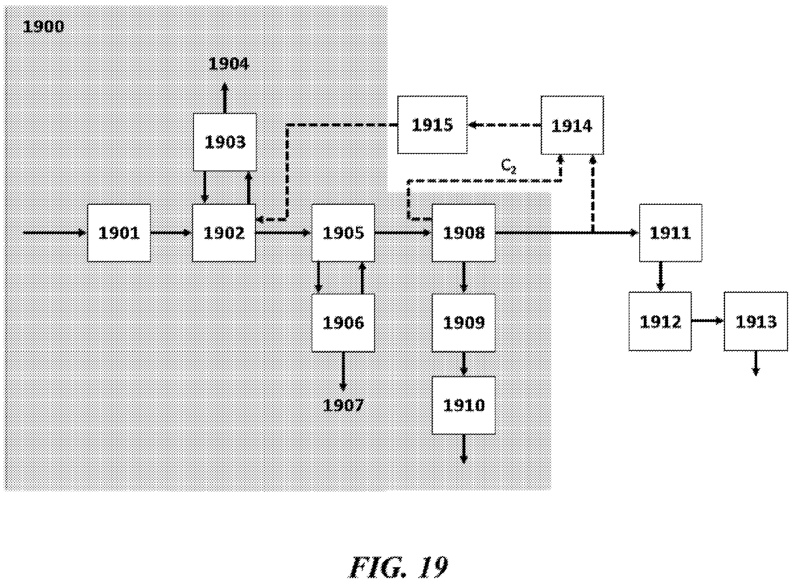

**FIG. 19** shows an integration of OCM and ETL processes with an LNG process for fuel production. A feed gas preparation system 1900 (e.g., of a gas processing plant) receives gas into its inlet gas facilities 1901. The stream is then directed into a gas treating unit 1902 with a solvent regeneration unit 1903, from which CO₂ and H₂S 1904 are recovered. The stream is then directed into a dehydration unit 1905 with a regeneration unit 1906, from which water 1907 is recovered. The stream is then directed to a liquid petroleum gas (LPG) extraction unit 1908, from which condensate is recovered and sent to storage 1909 (e.g., LPG/C₅₊ storage) and offloading 1910 for transportation (e.g., on ships). Dry gas from the LPG extraction unit is directed to an LNG liquefaction unit 1911, from which LNG product is directed to storage 1912 and offloading 1913 for transportation (e.g., on ships). At least a portion of the dry gas and C₂ from the LPG extraction unit can be directed to an OCM unit 1914. The OCM product stream can be directed to an ETL unit 1915, with fuel products (e.g., high octane gasoline) mixed with condensate from the LNG process or separated via a dedicated column. Light products and unreacted methane, for example, can be directed back into the gas treatment unit.

LNG processes can also be integrated with OCM processes for polymer production. For example, methane and ethane can be converted to a polymer (e.g., polyethylene). Capital expenditure (CapEx) can be reduced due to synergies and overlap in needed equipment, such as product separations equipment. The value of the polymer produced can be used to pay for the OCM processes, the polymerization process, and to offset the cost of an LNG process, for example.

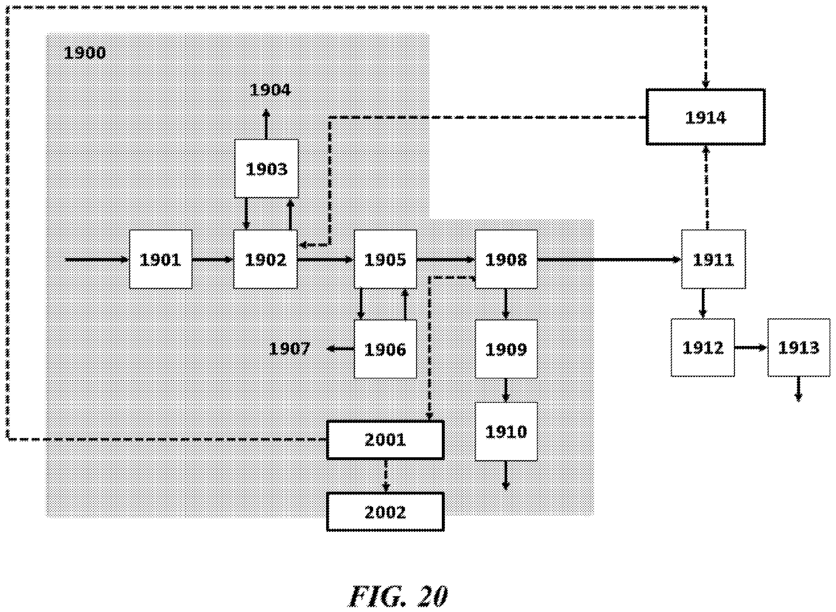

**FIG. 20** shows an integration of an OCM process with an LNG process for polymer production. C₂ compounds from the LPG extraction unit are directed to a C₂ splitter 2001. Ethylene from the C₂ splitter is directed into a polyethylene unit 2002, while ethane from the C₂ splitter is directed to the OCM unit.

### Integration of OCM Processes with an Oxalic Acid/Oxalate Process

An OCM process can be integrated with production of oxalic acid, oxalates, or derivatives thereof. For example, CO₂ produced in an OCM process can be directed to a reactor (e.g., an electrochemical reactor) for use in oxalic acid or oxalate production. Clean CO₂ from OCM can be converted to oxalate or oxalic acid, and optionally further to derivatives including glycolic acid, ethylene glycol, diglycolic acid, nitriloacetic acid, glyoxylic acid, and acetic acid.

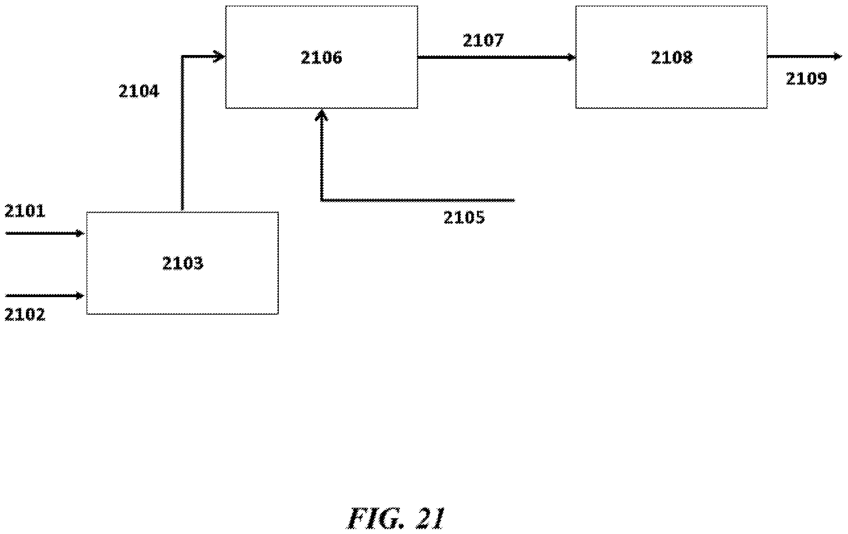

**FIG. 21** shows an exemplary schematic for integration of OCM with oxalic acid or oxalate production. Methane (e.g., natural gas) 2101 and oxygen 2102 are directed into an OCM unit 2103. CO₂ 2104 from the OCM unit and hydrogen 2105 are directed into a reactor (e.g., electrocatalytic/electrochemical reductive coupling of CO₂ reactor) to produce oxalic acid and/or oxalates 2107. The oxalic acid and/or oxalates can be directed into a hydrogenation reactor 2108 to produce other derivative products 2109.

### Integration of OCM Processes with an Ethylene Glycol Process

An OCM process can be integrated with production of ethylene glycol. For example, ethylene produced in an OCM process can be directed to a reactor (e.g., an oxidation reactor) for use in ethylene oxide production. Ethylene oxide can then be converted further to derivatives including ethylene glycol.

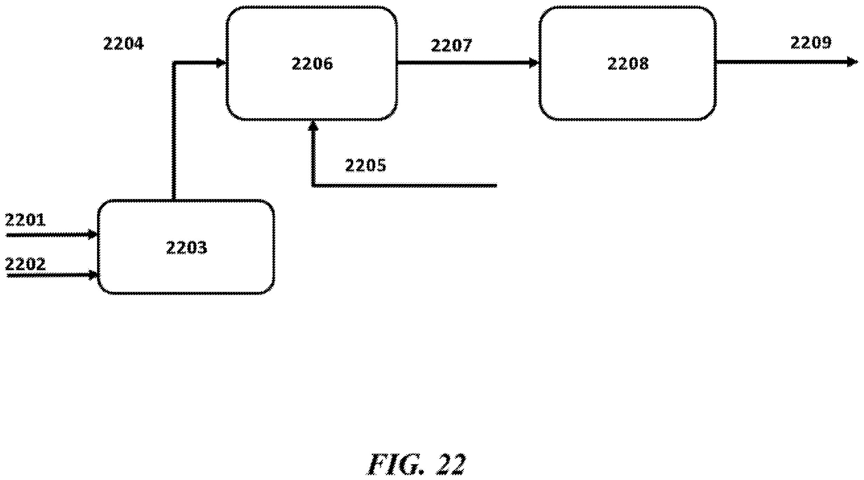

**FIG. 22** shows an exemplary schematic for integration of OCM with ethylene glycol production. Methane (e.g., natural gas) 2201 and oxygen 2202 are directed into an OCM unit 2203 to produce ethylene 2204. The ethylene and oxygen 2205 (e.g., air or pure oxygen) are directed into an oxidation reactor 2206, which produces ethylene oxide 2207. The ethylene oxide is then directed into a hydration reactor 2208 to produce ethylene glycol 2209.

### Integration of OCM Processes with a Propylene Process

OCM processes can be integrated with processes for the production of propylene, such at metathesis processes. Metathesis units can convert butene-2 and ethylene into propylene. The propylene produced can be of polymer grade and used as a feedstock to produce polypropylene.

The metathesis reaction can utilize an ethylene feed and a C₄ olefinic feed to produce propylene via a disproportionation reaction. In the absence of a C₄ feed, ethylene can be dimerized to produce the C₄ olefins used for metathesis. The C₄ olefin can be a butene-2 rich stream where the butene-2 content can be greater than about 90%, greater than about 93%, greater than about 95%, greater than about 97% or greater than about 99%. An OCM module can provide ethylene (e.g., polymer grade) to a dimerization unit, and/or to a metathesis unit. The metathesis reactor may contain a section for isomerization of butene-1 to butene-2. The product from the metathesis unit can contain predominantly propylene (and varying amounts of unreacted ethylene and butenes), along with some heavy C₅₊ components. Conventional metathesis units can include C₂ separation, C₃ separation and a de-oiler (C₅₊ removal). A metathesis unit integrated with an OCM system can have a common separations and purification system where the product stream from the metathesis unit is routed to the C₂ separations section of the OCM module (de-ethanizer). The de-ethanizer overhead can be sent to the C₂ splitter to generate polymer grade ethylene and an ethane product. The ethane product can be recycled to the OCM reactor. A part of the ethylene produced can be sent to the dimerization reactor and the remaining ethylene is sent to the metathesis unit. The de-ethanizer bottoms stream can be sent to a de-propanizer, followed by a C₃ splitter to produce (polymer grade) propylene. The de-propanizer bottoms can be sent to a de-butanizer or a de-pentanizer to recover a C₄ raffinate. In some embodiments, the butene rich stream from dimerization reactor can be isomerized in a reactive distillation section to convert butene-1 to butene-2 and separate the butene-2 for the metathesis reactor.

In some embodiments, the C₄ rich stream can be sourced from a refinery or a steam cracker. The refinery or steam cracker C₄ stream can be sufficient to provide for the metathesis unit with no dimerization required. In some cases, the C₄ stream can be mixed with the C₄ stream from the dimerization reactor. In either case (i.e., dimerization alone, dimerization plus off gas recovery or only off gas processing), the C₄ processing can also include either a selective hydrogenation unit (SHU) to hydrogenate any C₄ dienes to olefins, or a butadiene recovery unit or a total hydrogenation unit to hydrogenate the remaining C₄s after butene-2 has been utilized. In some cases, the final product is a C₄ LPG/ C₄ raffinate containing butanes, and unreacted butenes.

The integrations described herein (e.g., OCM + metathesis + polypropylene) can yield many advantages from a process and economic standpoint. The combined system can have a common separations and recovery system, a common refrigeration system, and take advantage of an integrated site with respect to utilities and off-sites. Additionally, the OCM system can generate excess steam for the entire system.

Additionally, ethylene from an OCM process can be supplied as a co-monomer for polypropylene production (e.g., 8-15% ethylene co-monomer). A separations section of an OCM process can handle the recycle streams from a metathesis unit and a polypropylene unit in addition to the separations for the OCM process itself.

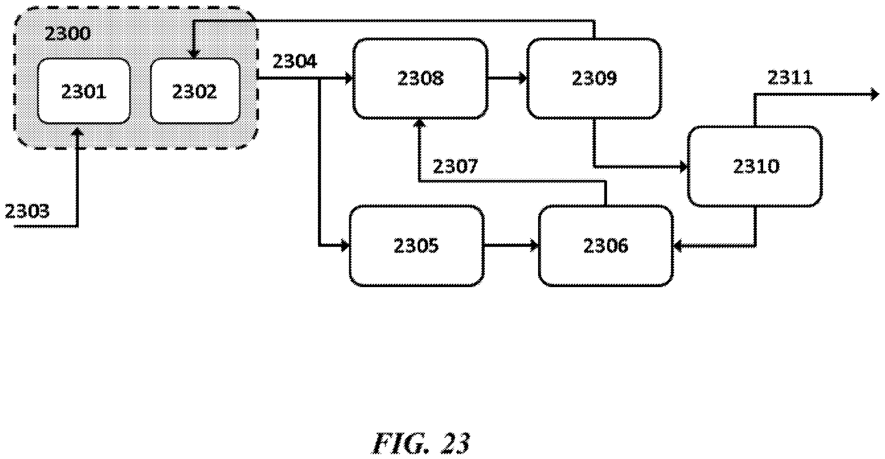

For example, **FIG. 23** shows an exemplary schematic for integration of OCM with metathesis for propylene production. An OCM unit 2300 with an OCM reactor 2301 and a separations section 2302 receives a methane stream 2303 (e.g., natural gas) and produces an ethylene product stream 2304 (e.g., polymer-grade ethylene). A portion of the ethylene stream can be directed into a dimerization reactor 2305 to produce C₄ products, which can then be separated in a C₄ separation unit 2306. Butene-2 2307 from the C₄ separation unit can be directed into a metathesis reactor 2308 along with ethylene from the OCM unit. The metathesis product stream can be directed to a C₂ separation unit 2309, with C₂ compounds being sent as a recycle stream to the OCM unit separations section. C₃₊ compounds can be directed from the C₂ separations unit to a C₃ separations unit 2310. Propylene 2311 can be recovered from the C₃ separations unit, with C₄₊ compounds directed to the C₄ separation unit.

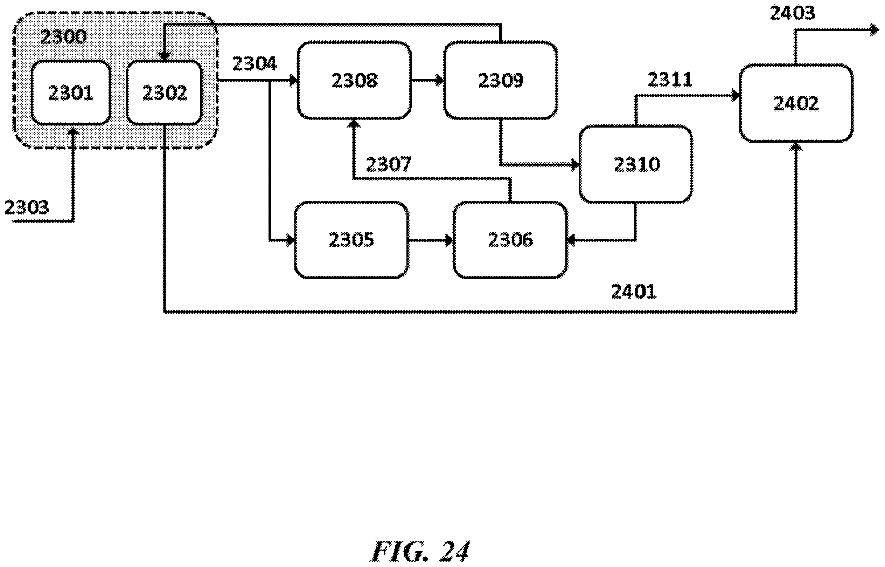

Propylene can be further processed into polypropylene. For example, **FIG. 24** shows the propylene 2311 being directed, along with ethylene co-monomer 2401 from the OCM unit, into a polypropylene unit 2402 to produce polypropylene 2403.

Metathesis can be conducted as a vapor phase equilibrium reaction. Metathesis can achieve n-butene conversion of about 72% single pass and about 90%-95% overall conversion. The reaction can be conducted at isothermal or nearly isothermal conditions, and can be energy neutral. The presence of iso-butene can lead to more side reactions producing 2,3-dimethylbutene and isoamylene.

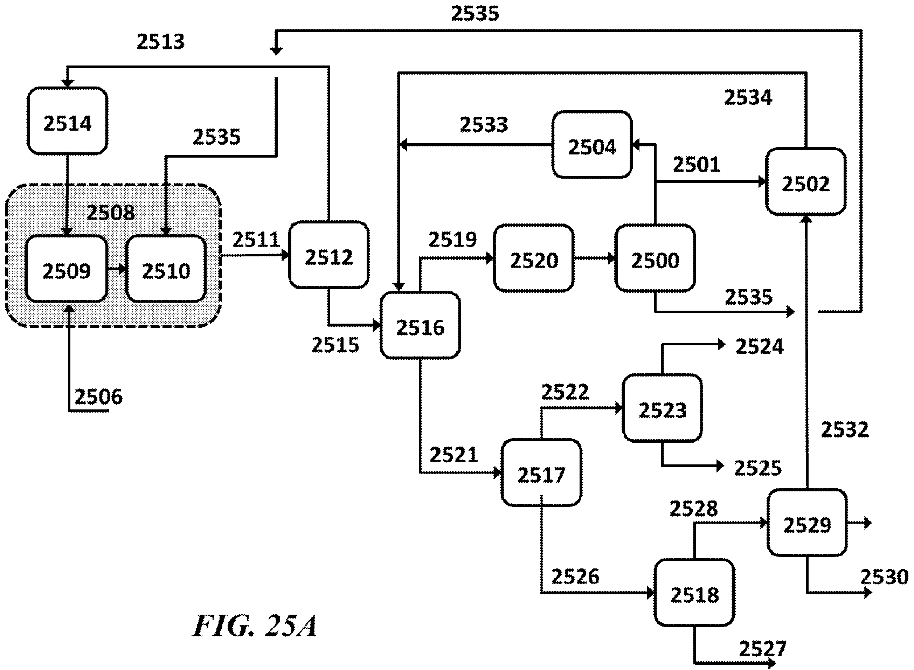

In some cases, the recovery systems are integrated. For example, with reference to **FIG. 25A****,** a case is shown having a C₂ splitter **2500** that produces enriched ethylene **2501** for the metathesis unit **2502** and/or the dimerization unit **2504.** In some cases, the enriched ethylene is polymer-grade ethylene (which can also be used as a co-monomer in the production of polypropylene). In some instances, the C₂ splitter **2500** is not operated at conditions that result in polymer-grade ethylene. The enriched ethylene stream can be about 60%, about 70%, about 80%, about 90%, about 95%, or about 99% ethylene by mass. In some cases, the enriched ethylene stream is at least about 60%, at least about 70%, at least about 80%, at least about 90%, at least about 95%, or at least about 99% ethylene by mass.

Continuing with **FIG. 25A****,** reactants **2506** (i.e., methane and O₂) can be fed into an OCM reactor **2508** having a catalyst bed **2509** and an ethane conversion section **2510.** The OCM reactor can produce an OCM effluent **2511** that goes to a de-methanizer **2512.** In some cases, there are additional units in the OCM process that are not shown, such as compressors, CO₂ removal units, drying units, desulfurization units, quenchers and heat exchangers. The demethnizer overhead **2513** can contain C₁ compounds and go to a methanation unit **2514** for conversion into methane and recycle to the OCM reactor **2508.** As used herein, the terms "overhead" and "bottoms" do not limit the portion or section of the separation column from which the stream emerges (e.g., in some cases, the "bottoms" can come out of the middle or top of the separation column).

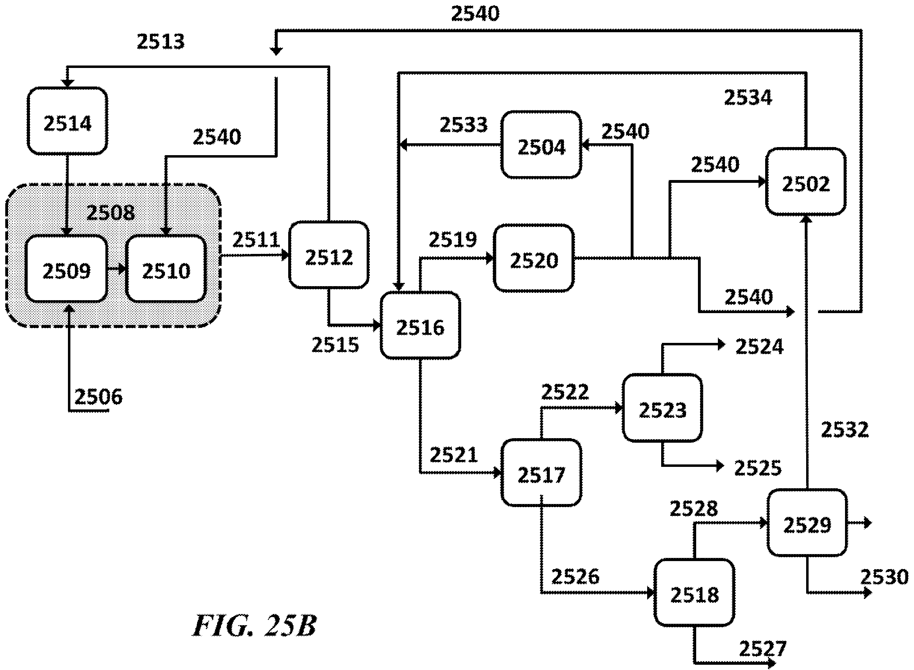

The de-methanizer bottoms **2515** can include C₂₊ compounds and continue into a fractionation train including a de-ethanizer **2516**, a de-propanizer **2517** and a de-butanizer **2518**. The de-ethanizer overhead **2519** can contain C₂ compounds and go to a hydrogenation unit **2520**, which hydrogenation unit can (selectively) hydrogenate acetylene. As described herein, the C₂ compounds can be separated into an enriched ethylene stream (i.e., using the C₂ splitter **2500**), or not separated as shown in **FIG. 25B**.

The de-ethanizer bottoms **2521** can contain C₃₊ compounds and be taken to the de-propanizer **2517.** The de-propanizer overhead **2522** can contain C₃ compounds that can be split in a C₃ splitter **2523** into propane **2524** and propylene **2525.** In some cases, the propylene is polymer-grade. In some cases, the propylene is used to make polypropylene (optionally with an ethylene co-monomer, such as derived from the present process, i.e., from the C₂ splitter **2500**). In some embodiments, the propylene **2525** is about 85%, about 90%, about 95%, about 99%, about 99.5%, about 99.9%, or about 99.95% pure. In some instances, the propylene **2525** is at least about 85%, at least about 90%, at least about 95%, at least about 99%, at least about 99.5%, at least about 99.9%, or at least about 99.95% pure.

The de-propanizer bottoms **2526** can contain C₄₊ compounds and be directed to a de-butanizer **2518.** The de-butanizer can produce a bottoms stream **2527** that includes C₅₊ compounds and an overhead stream **2528** comprising C₄ compounds, which C₄ compounds can be sent to a C₄ splitter **2529**. The C₄ splitter can produce a plurality of streams (i.e., **2530, 2531** and **2532**) including a stream enriched in butene-2 **2532**. In some embodiments, the butene-2 **2532** is about 85%, about 90%, about 95%, about 99%, about 99.5%, about 99.9%, or about 99.95% pure. In some instances, the butene-2 **2532** is at least about 85%, at least about 90%, at least about 95%, at least about 99%, at least about 99.5%, at least about 99.9%, or at least about 99.95% pure. The butene-2 **2532** can go to the metathesis unit **2502**.

Additional butene-2 **2533** can be produced from the dimerization module **2504** (i.e., from ethylene). The additional butene-2 **2533** can be used directly in the metathesis reactor **2502** in some cases. However, as shown here, the additional butene-2 can be recycled to the fractionation train (e.g., to the de-ethanizer **2516**) to enrich the concentration of butene-2 prior to metathesis.

The metathesis unit can produce a propylene stream **2534** that can be utilized directly or enriched (e.g., to polymer grade propylene) by recycling the dilute propylene stream **2534** to the fractionation train (e.g., to the de-ethanizer **2516**).

The process can produce a number of additional streams that can be utilized directly or recycled in the process, such as an ethane stream **2535** coming from the C₂ splitter that can be recycled to the catalyst bed **2509** and/or ethane conversion section **2510** of the OCM reactor **2508**.

In some cases, the C₂ compounds are not split into enriched ethylene or enriched ethane streams. With reference to **FIG. 25B**, the de-ethanizer overhead **2519** can be used in the metathesis module **2502**, in the dimerization module **2504**, and/or can be recycled to the OCM reactor **2508** directly (e.g., without first being separated in a C₂ splitter). In some cases, the C₂ stream **2519** can go through a hydrogenation unit **2520** (e.g., that hydrogenates acetylene) to produce a hydrogenated C₂ stream **2540**, which hydrogenated C₂ stream **2540**can be used in the metathesis module **2502**, in the dimerization module **2504**. In some embodiments, the hydrogenated C₂ stream **2540** can contain about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% compounds other than ethylene. In some cases, the hydrogenated C₂ stream **2540** can contain at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, or at least about 95% compounds other than ethylene.

It should be understood from the foregoing that, while particular implementations have been illustrated and described, various modifications can be made thereto and are contemplated herein. It is also not intended that the invention be limited by the specific examples provided within the specification. While the invention has been described with reference to the aforementioned specification, the descriptions and illustrations of the preferable embodiments herein are not meant to be construed in a limiting sense. Furthermore, it shall be understood that all aspects of the invention are not limited to the specific depictions, configurations or relative proportions set forth herein which depend upon a variety of conditions and variables. Various modifications in form and detail of the embodiments of the invention will be apparent to a person skilled in the art. It is intended that the following claims define the scope of the invention and that methods and structures within the scope of these claims be covered thereby.

## Claims

1. A method for producing propylene, comprising:
(a) directing methane (CH₄) and oxygen (O₂) into an oxidative coupling of methane (OCM) reactor that reacts the CH₄ and the O₂ to yield an OCM product stream comprising hydrocarbon compounds containing at least two carbon atoms (C₂₊ compounds) including ethylene;
(b) directing the OCM product stream into a separations unit that yields an ethylene stream comprising ethylene from the OCM product stream;
(c) directing a first portion of ethylene from the ethylene stream into a dimerization reactor that reacts the ethylene in a dimerization reaction to yield a butene stream comprising butene compounds;
(d) directing the butene stream into a C₄ separations unit that yields a butene-2 stream comprising butene-2 from the butene stream; and
(e) directing the butene-2 stream and a second portion of ethylene from the ethylene stream into a metathesis reactor that reacts the butene-2 and the ethylene to yield a metathesis product stream comprising C₂₊ compounds including propylene.

2. The method of claim 1, further comprising directing the metathesis product stream into a C₂ separations unit that separates the metathesis product stream to yield a C₂ stream comprising C₂ compounds and a C₃₊ stream comprising C₃₊ compounds including propylene.

3. The method of claim 2, further comprising directing the C₂ stream into the separations unit.

4. The method of claim 2, further comprising directing the C₃₊ stream into a C₃ separations unit that separates the C₃₊ stream to yield a C₃ stream comprising propylene and a C₄₊ stream comprising C₄₊ products.

5. The method of claim 4, further comprising directing the C₄₊ stream into the C₄ separations unit.

6. The method of claim 2, further comprising directing the propylene from the metathesis product stream into a polypropylene unit that reacts the propylene to yield a polypropylene product stream comprising polypropylene.

7. The method of claim 6, further comprising directing ethylene from the separations unit to the polypropylene unit, wherein the polypropylene unit reacts the ethylene as a co-monomer with the propylene.

8. The method of claim 7, wherein the ratio of ethylene co-monomer to total monomer and co-monomer is from about 0.01:0.99 to about 0.15:0.85

9. The method of claim 7, wherein the ratio of ethylene co-monomer to total monomer and co-monomer is from about 0.08:0.92 to about 0.15:0.85.

10. The method of claim 1 where step (a) further comprises directing ethane (C₂H₆) into the OCM reactor.

11. A system for producing propylene, comprising:
an oxidative coupling of methane (OCM) reactor that receives methane (CH₄) and oxygen (O₂) and reacts the CH₄ and the O₂ to yield an OCM product stream comprising hydrocarbon compounds containing at least two carbon atoms (C₂₊ compounds) including ethylene;
a separations unit that receives the OCM product stream and yields an ethylene stream comprising ethylene from the OCM product stream;
a dimerization reactor that receives a first portion of ethylene from the ethylene stream and reacts the ethylene in a dimerization reaction to yield a butene stream comprising butene compounds;
a C₄ separations unit that receives the butene stream and yields a butene-2 stream comprising butene-2 from the butene stream; and
a metathesis reactor that receives the butene-2 stream and a second portion of ethylene from the ethylene stream and reacts the butene-2 and the ethylene to yield a metathesis product stream comprising C₂₊ compounds including propylene.

12. The system of claim 11, further comprising a C₂ separations unit that receives the metathesis product stream and separates the metathesis product stream to yield a C₂ stream comprising C₂ compounds and a C₃₊ stream comprising C₃₊ compounds including propylene.

13. The system of claim 12, wherein the separations unit receives the C₂ stream.

14. The system of claim 12, further comprising a C₃ separations unit that receives the C₃₊ stream and separates the C₃₊ stream to yield a C₃ stream comprising propylene and a C₄₊ stream comprising C₄₊ products.

15. The system of claim 14, further comprising a polypropylene unit that receives the propylene from the metathesis product stream and reacts the propylene to yield a polypropylene product stream comprising polypropylene.

## Patentansprüche

1. Ein Verfahren zum Produzieren von Propylen, beinhaltend:
(a) Leiten von Methan (CH₄) und Sauerstoff (O₂) in einen Reaktor zum oxidativen Koppeln von Methan (OCM), der das CH₄ und den O₂ umsetzt, um einen OCM-Produktstrom zu ergeben, der Kohlenwasserstoffverbindungen, die mindestens zwei Kohlenstoffatome (C₂+-Verbindungen) enthalten, einschließlich Ethylen, beinhaltet;
(b) Leiten des OCM-Produktstroms in eine Trenneinheit, die einen Ethylenstrom ergibt, der Ethylen aus dem OCM-Produktstrom beinhaltet;
(c) Leiten eines ersten Anteils von Ethylen aus dem Ethylenstrom in einen Dimerisierungsreaktor, der das Ethylen in einer Dimerisierungsreaktion umsetzt, um einen Butenstrom zu ergeben, der Butenverbindungen beinhaltet;
(d) Leiten des Butenstroms in eine C₄-Trenneinheit, die einen Buten-2-Strom ergibt, der Buten-2 aus dem Butenstrom beinhaltet; und
(e) Leiten des Buten-2-Stroms und eines zweiten Anteils von Ethylen aus dem Ethylenstrom in einen Metathesereaktor, der das Buten-2 und das Ethylen umsetzt, um einen Metatheseproduktstrom, der C₂₊-Verbindungen beinhaltet, einschließlich Propylen, zu ergeben.

2. Verfahren gemäß Anspruch 1, ferner beinhaltend Leiten des Metatheseproduktstroms in eine C₂-Trenneinheit, die den Metatheseproduktstrom trennt, um einen C₂-Strom, der C₂-Verbindungen beinhaltet, und einen C₃₊-Strom, der C₃₊-Verbindungen, einschließlich Propylen, beinhaltet, zu ergeben.

3. Verfahren gemäß Anspruch 2, ferner beinhaltend das Leiten des C₂-Stroms in die Trenneinheit.

4. Verfahren gemäß Anspruch 2, ferner beinhaltend das Leiten des C₃₊-Stroms in eine C₃-Trenneinheit, die den C₃₊-Strom trennt, um einen C₃-Strom, der Propylen beinhaltet, und einen C₄₊-Strom, der C₄₊-Produkte beinhaltet, zu ergeben.

5. Verfahren gemäß Anspruch 4, ferner beinhaltend das Leiten des C₄₊-Stroms in die C₄-Trenneinheit.

6. Verfahren gemäß Anspruch 2, ferner beinhaltend das Leiten des Propylens aus dem Metatheseproduktstrom in eine Polypropyleneinheit, die das Propylen umsetzt, um einen Polypropylenproduktstrom zu ergeben, der Polypropylen beinhaltet.

7. Verfahren gemäß Anspruch 6, ferner beinhaltend das Leiten von Ethylen aus der Trenneinheit zu der Polypropyleneinheit, wobei die Polypropyleneinheit das Ethylen als ein Comonomer mit dem Propylen umsetzt.

8. Verfahren gemäß Anspruch 7, wobei das Verhältnis von Ethylen-Comonomer zu Gesamtmonomer und Comonomer von etwa 0,01 : 0,99 bis etwa 0,15 : 0,85 beträgt.

9. Verfahren gemäß Anspruch 7, wobei das Verhältnis von Ethylen-Comonomer zu Gesamtmonomer und Comonomer von etwa 0,08 : 0,92 bis etwa 0,15 : 0,85 beträgt.

10. Verfahren gemäß Anspruch 1, wobei Schritt (a) ferner das Leiten von Ethan (C₂H₆) in den OCM-Reaktor beinhaltet.

11. Ein System zum Produzieren von Propylen, beinhaltend:
einen Reaktor zum oxidativen Koppeln von Methan (OCM), der Methan (CH₄) und Sauerstoff (O₂) aufnimmt und das CH₄ und den O₂ umsetzt, um einen OCM-Produktstrom zu ergeben, der Kohlenwasserstoffverbindungen, die mindestens zwei Kohlenstoffatome (C₂₊-Verbindungen) enthalten, einschließlich Ethylen, beinhaltet;
eine Trenneinheit, die den OCM-Produktstrom aufnimmt und einen Ethylenstrom ergibt, der Ethylen aus dem OCM-Produktstrom beinhaltet;
einen Dimerisierungsreaktor, der einen ersten Anteil von Ethylen aus dem Ethylenstrom aufnimmt und das Ethylen in einer Dimerisierungsreaktion umsetzt, um einen Butenstrom zu ergeben, der Butenverbindungen beinhaltet;
eine C₄-Trenneinheit, die den Butenstrom aufnimmt und einen Buten-2-Strom ergibt, der Buten-2 aus dem Butenstrom beinhaltet; und
einen Metathesereaktor, der den Buten-2-Strom und einen zweiten Anteil von Ethylen aus dem Ethylenstrom aufnimmt und das Buten-2 und das Ethylen umsetzt, um einen Metatheseproduktstrom, der C₂₊-Verbindungen beinhaltet, einschließlich Propylen, zu ergeben.

12. System gemäß Anspruch 11, ferner beinhaltend eine C₂-Trenneinheit, die den Metatheseproduktstrom aufnimmt und den Metatheseproduktstrom trennt, um einen C₂-Strom, der C₂-Verbindungen beinhaltet, und einen C₃₊-Strom zu ergeben, der C₃₊-Verbindungen, einschließlich Propylen, beinhaltet.

13. System gemäß Anspruch 12, wobei die Trenneinheit den C₂-Strom aufnimmt.

14. System gemäß Anspruch 12, ferner beinhaltend eine C₃-Trenneinheit, die den C₃₊-Strom aufnimmt und den C₃₊-Strom trennt, um einen C₃-Strom, der Propylen beinhaltet, und einen C₄₊-Strom zu ergeben, der C₄₊-Produkte beinhaltet.

15. System gemäß Anspruch 14, ferner beinhaltend eine Polypropyleneinheit, die das Propylen aus dem Metatheseproduktstrom aufnimmt und das Propylen umsetzt, um einen Polypropylenproduktstrom zu ergeben, der Polypropylen beinhaltet.

## Revendications

1. Un procédé pour produire du propylène, comprenant :
(a) le fait de diriger du méthane (CH₄) et de l'oxygène (O₂) jusque dans un réacteur de couplage oxydant du méthane (OCM, pour *oxidative coupling of methane*) qui fait réagir le CH₄ et l'O₂ afin de donner un courant de produit d'OCM comprenant des composés hydrocarbures contenant au moins deux atomes de carbone (composés en C₂₊) incluant de l'éthylène ;
(b) le fait de diriger le courant de produit d'OCM jusque dans une unité de séparation qui donne un courant d'éthylène comprenant de l'éthylène provenant du courant de produit d'OCM ;
(c) le fait de diriger une première portion d'éthylène provenant du courant d'éthylène jusque dans un réacteur de dimérisation qui fait réagir l'éthylène dans une réaction de dimérisation afin de donner un courant de butène comprenant des composés de butène ;
(d) le fait de diriger le courant de butène jusque dans une unité de séparation de C₄ qui donne un courant de butène-2 comprenant du butène-2 provenant du courant de butène ; et
(e) le fait de diriger le courant de butène-2 et une deuxième portion d'éthylène provenant du courant d'éthylène jusque dans un réacteur de métathèse qui fait réagir le butène-2 et l'éthylène afin de donner un courant de produit de métathèse comprenant des composés en C₂₊ incluant du propylène.

2. Le procédé de la revendication 1, comprenant en outre le fait de diriger le courant de produit de métathèse jusque dans une unité de séparation de C₂ qui sépare le courant de produit de métathèse afin de donner un courant de C₂ comprenant des composés en C₂ et un courant de Cₐ₊ comprenant des composés en C₃₊ incluant du propylène.

3. Le procédé de la revendication 2, comprenant en outre le fait de diriger le courant de C₂ jusque dans l'unité de séparation.

4. Le procédé de la revendication 2, comprenant en outre le fait de diriger le courant de C₃₊ jusque dans une unité de séparation de C₃ qui sépare le courant de C₃₊ afin de donner un courant de C₃ comprenant du propylène et un courant de C₄₊ comprenant des produits en C₄₊.

5. Le procédé de la revendication 4, comprenant en outre le fait de diriger le courant de C₄₊ jusque dans l'unité de séparation de C₄.

6. Le procédé de la revendication 2, comprenant en outre le fait de diriger le propylène provenant du courant de produit de métathèse jusque dans une unité de polypropylène qui fait réagir le propylène afin de donner un courant de produit de polypropylène comprenant du polypropylène.

7. Le procédé de la revendication 6, comprenant en outre le fait de diriger de l'éthylène provenant de l'unité de séparation vers l'unité de polypropylène, dans lequel l'unité de polypropylène fait réagir l'éthylène en tant que co-monomère avec le propylène.

8. Le procédé de la revendication 7, dans lequel le rapport de co-monomère éthylène à un total de monomère et co-monomère va d'environ 0,01/0,99 à environ 0,15/0,85.

9. Le procédé de la revendication 7, dans lequel le rapport de co-monomère éthylène à un total de monomère et co-monomère va d'environ 0,08/0,92 à environ 0,15/0,85.

10. Le procédé de la revendication 1 où l'étape (a) comprend en outre le fait de diriger de l'éthane (C₂H₆) jusque dans le réacteur d'OCM.

11. Un système pour produire du propylène, comprenant :
un réacteur de couplage oxydant du méthane (OCM) qui reçoit du méthane (CH₄) et de l'oxygène (O₂) et fait réagir le CH₄ et l'O₂ afin de donner un courant de produit d'OCM comprenant des composés hydrocarbures contenant au moins deux atomes de carbone (composés en C₂₊) incluant de l'éthylène ;
une unité de séparation qui reçoit le courant de produit d'OCM et donne un courant d'éthylène comprenant de l'éthylène provenant du courant de produit d'OCM ;
un réacteur de dimérisation qui reçoit une première portion d'éthylène provenant du courant d'éthylène et fait réagir l'éthylène dans une réaction de dimérisation afin de donner un courant de butène comprenant des composés de butène ;
une unité de séparation de C₄ qui reçoit le courant de butène et donne un courant de butène-2 comprenant du butène-2 provenant du courant de butène ; et
un réacteur de métathèse qui reçoit le courant de butène-2 et une deuxième portion d'éthylène provenant du courant d'éthylène et fait réagir le butène-2 et l'éthylène afin de donner un courant de produit de métathèse comprenant des composés en C₂₊ incluant du propylène.

12. Le système de la revendication 11, comprenant en outre une unité de séparation de C₂ qui reçoit le courant de produit de métathèse et sépare le courant de produit de métathèse afin de donner un courant de C₂ comprenant des composés en C₂ et un courant de C₃₊ comprenant des composés en C₃₊ incluant du propylène.

13. Le système de la revendication 12, dans lequel l'unité de séparation reçoit le courant de C₂.

14. Le système de la revendication 12, comprenant en outre une unité de séparation de C₃ qui reçoit le courant de C₃₊ et sépare le courant de C₃₊ afin de donner un courant de C₃ comprenant du propylène et un courant de C₄₊ comprenant des produits en C₄+.

15. Le système de la revendication 14, comprenant en outre une unité de polypropylène qui reçoit le propylène provenant du courant de produit de métathèse et fait réagir le polypropylène afin de donner un courant de produit de polypropylène comprenant du polypropylène.
